(19) Europäisches Patentamt
European Patent Office
Office européen des brevets

(11) Veröffentlichungsnummer: **0 213 571**
**A2**

(12) **EUROPÄISCHE PATENTANMELDUNG**

(21) Anmeldenummer: **86111625.9**

(22) Anmeldetag: **22.08.86**

(51) Int. Cl.⁴: **C07D 207/335**, C07D 209/14, C07D 401/00, C07D 403/00, C07D 405/00, C07D 409/00, C07D 417/00, A61K 31/40

(30) Priorität: **04.09.85 DE 3531504**

(43) Veröffentlichungstag der Anmeldung: **11.03.87 Patentblatt 87/11**

(84) Benannte Vertragsstaaten: **AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Geiss, Karl-Heinz, Dr.**
**Kirchenstrasse 8**
**D-6711 Beindersheim(DE)**
Erfinder: **Himmele, Walter, Dr.**
**Eichenweg 14**
**D-6909 Walldorf(DE)**
Erfinder: **Degner, Dieter, Dr.**
**Kurpfalzstrasse 8**
**D-6701 Dannstadt-Schauernheim(DE)**
Erfinder: **Siegel, Hardo, Dr.**
**Hans-Purrmann-Allee 25**
**D-6720 Speyer(DE)**
Erfinder: **Gries, Josef, Dr.**
**Roemerweg 43**
**D-6706 Wachenheim(DE)**
Erfinder: **Lenke, Dieter, Prof.Dr.**
**Kekuleplatz 1**
**D-6700 Ludwigshafen(DE)**

(54) **3-Aminomethylpyrrol-1-yl-alkylamine und diese Verbindungen enthaltende therapeutische Mittel.**

(57) Verbindungen der allgemeinen Formel I

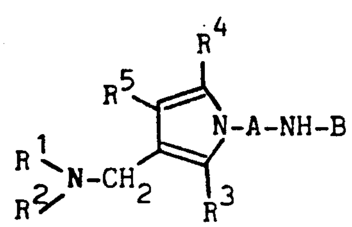

I,

worin

$R^1$ und $R^2$ unabhängig voneinander ein Wasserstoffatom oder einen $C_1$-$C_{12}$-Alkyl-, $C_3$-$C_{12}$-Alkenyl-oder $C_3$-$C_{12}$-Cycloalkylrest, die durch einen Phenyl-oder Naphthylrest, welche ihrerseits durch 1 oder 2 $C_{1-3}$-Alkyl-, $C_{1-3}$-Alkoxy-gruppen oder durch 1 bis 2 Fluor-, Chlor-oder Bromatome oder durch eine

EP 0 213 571 A2

Trifluormethyl-, Carboxy-, ($C_{1-5}$-Alkoxy)-carbonyl-, Cyan-oder $C_{1-4}$-Acylamidogruppe substituiert sein können, oder durch einen 5gliedrigen aromatischen Ring mit 1 bis 3 Stickstoffatomen oder einem Sauerstoff-oder Schwefelatom und ggf. 1-2 Stickstoffatomen, oder durch einen 6gliedrigen aromatischen Ring mit 1 bis 3 Stickstoffatomen, wobei diese heteroaromatischen Ringe ggf. 1 bis 3 $C_{1-3}$-Alkylgruppen tragen, substituiert sein können, bedeuten, oder $R^1$ und $R^2$ zusammen mit dem Stickstoffatom einen gesättigten heterocyclischen Ring mit 4 bis 12 Ringgliedern bilden, der durch eine Phenyl-und/oder eine Hydroxy-, eine $C_{1-4}$-Acyl-, eine ($C_{1-5}$-Alkoxy)-carbonyl-, eine Nitril-, eine N-Phenyl-N-($C_{1-4}$-Acyl)-amino-, eine N-Benzamidogruppe oder einen Benzimidazolin-2-on-1-yl-rest und/oder durch 1 bis 3 $C_{1-4}$-Alkylgruppen substituiert sein oder einen ankondensierten Benzolring tragen kann,

$R^3$, $R^4$ und $R^5$ unabhängig voneinander Wasserstoffatome oder $C_{1-3}$-Alkylgruppen bedeuten, wobei $R^4$ und $R^5$ gemeinsam mit der Doppelbindung auch einen Benzolring bedeuten können,

A einen $C_{2-8}$-Alkylenrest bedeutet, der einfach olefinisch oder acetylenisch ungesättigt sein kann und in dem ein Kohlenstoffatom durch ein Sauerstoff-, ein Schwefelatom oder eine Phenylengruppe ersetzt sein kann,

B eine der Gruppen $B^1$ bis $B^6$ bedeutet:

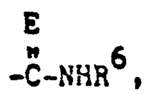 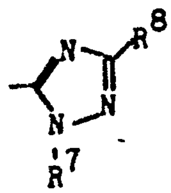 , 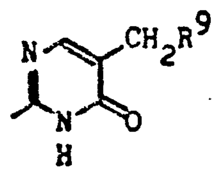

$B^1$ , $B^2$ $B^3$

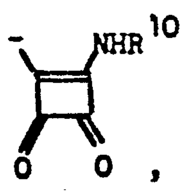 , 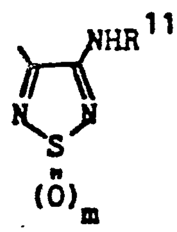 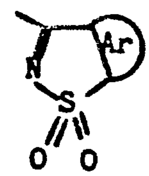

$B^4$ $B^5$ $B^6$

wobei

E S, $CH-NO_2$ oder N-CN bedeutet,

$R^6$ ein Wasserstoffatom oder einen $C_{1-3}$-Alkylrest, der durch einen 5gliedrigen aromatischen Ring mit 1 bis 3 Stickstoffatomen oder einem Sauerstoff-oder Schwefelatom und ggf. 1-2 Stickstoffatomen oder einen 6gliedrigen aromatischen Ring mit 1 bis 3 Stickstoffatomen, wobei diese heteroaromatischen Ringe ggf. 1 bis 3 $C_{1-3}$-Alkylgruppen tragen, oder durch eine Hydroxy-oder $C_{1-4}$ -Alkoxygruppe substituiert sein kann, bedeutet,

$R^7$ ein Wasserstoffatom oder einen $C_{1-3}$-Alkylrest bedeutet,

$R^8$ eine Aminogruppe oder den Rest $CH_2OR^{12}$ bedeutet, wobei

$R^{12}$ für ein Wasserstoffatom, einen $C_{1-3}$-Alkylrest, eine $C_{1-6}$-Acylgruppe, die Benzoylgruppe, den Tetrahydrofuran-2-yl oder Tetrahydropyran-2-yl-rest steht,

$R^9$ einen $C_{1-6}$-Alkylrest, eine Phenylgruppe, die durch je 1 oder 2 $C_{1-3}$-Alkyl-, $C_{1-3}$-Alkoxygruppen oder Chlor-oder Bromatome oder durch einen Methylendioxorest substituiert sein kann, oder einen 5gliedrigen aromatischen Ring mit 1 bis 3 Stickstoffatomen oder einem Sauerstoff-oder Schwefelatom und ggf. 1-2 Stickstoffatomen oder einen 6gliedrigen aromatischen Ring mit 1 bis 3 Stickstoffatomen, wobei diese heteroaromatischen Ringe ggf. 1 bis 3 $C_{1-3}$-Alkylgruppen tragen, bedeutet,

$R^{10}$ und $R^{11}$ Wasserstoffatome oder $C_{1-3}$-Alkylreste, die durch eine Phenylgruppe, die ihrerseits mit je 1 oder 2 $C_{1-3}$-Alkyl-, $C_{1-3}$-Alkoxygruppen oder Chlor-oder Bromatomen substituiert sein kann, oder

durch einen 5gliedrigen aromatischen Ring mit 1 bis 3 Stickstoffatomen oder einem Sauerstoff-oder Schwefelatom und ggf. 1-2 Stickstoffatomen oder einem 6gliedrigen aromatischen Ring mit 1 bis 3 Stickstoffatomen, wobei diese heteroaromatischen Ringe ggf. 1 bis 3 $C_{1-3}$-Alkylgruppen tragen, oder durch eine Hydroxy-oder $C_{1-3}$-Alkoxygruppe substituiert sein können, bedeuten,

m die Zahl 0, 1 oder 2 bedeutet und

Ar einen ankondensierten Benzol-oder Thiophenring darstellt, sowie deren physiologisch verträglichen Salze,

und die Verbindungen oder deren Salze als Wirkstoff enthaltende therapeutische Mittel.

# 3-Aminomethylpyrrol-1-yl-alkylamine und diese Verbindungen enthaltende therapeutische Mittel

Die Erfindung betrifft 3-Aminomethyl-pyrrol-1-yl-alkylamine, ein Zwischenprodukt bei ihrer Herstellung, sowie diese Verbindungen enthaltende therapeutische Mittel. Insbesondere besitzen sie Histamin-$H_2$-Rezeptoren-antagonisierende Wirkungen.

Verbindungen, die die Wirkung von Histamin auf Histamin-$H_2$-Rezeptoren antagonisieren, können bei der Behandlung von Krankheiten wie Ulcus ventriculi, Ulcus duodeni oder Gastritis verwendet werden, in denen eine Erniedrigung der Magensäuresekretion die Heilung günstig beeinflußt. Außerdem können Verbindungen mit Histamin-$H_2$-Rezeptoren-blockierender Aktivität bei der Behandlung von durch Histamin hervorgerufenen allergischen und entzündlichen Zuständen eingesetzt werden, ggf. in Kombination mit Histamin-$H_1$-Rezeptorantagonisten.

Die beiden Verbindungen Cimetidin und Ranitidin sind die einzigen bisher im Markt eingeführten $H_2$-Rezeptorenblocker (Review: M. J. Daly, B. J. Price, Progress in Medicinal Chemistry, Vol. 20, S. 337 -368 1983)).

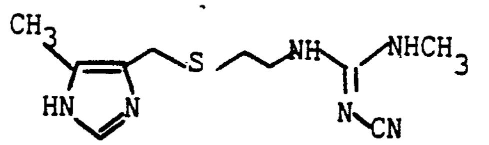
Cimetidin

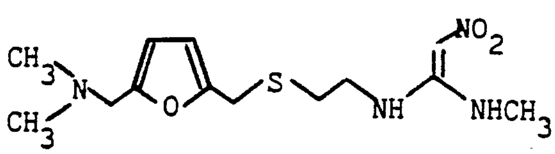
Ranitidin

Gegenstand der Erfindung sind Verbindungen der allgemeinen Formel I

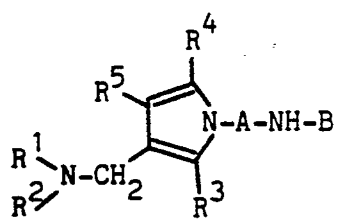
I,

worin

$R^1$ und $R^2$ unabhängig voneinander ein Wasserstoffatom oder einen $C_1$-$C_{12}$-Alkyl-, $C_3$-$C_{12}$-Alkenyl-oder $C_3$-$C_{12}$-Cycloalkylrest, die durch einen Phenyl-oder Naphthylrest, welche ihrerseits durch 1 oder 2 $C_{1-3}$ -Alkyl-, C $_{1-3}$-Alkoxy-gruppen oder durch 1 bis 2 Fluor-, Chlor-oder Bromatome oder durch eine Trifluormethyl-, Carboxy-, ($C_{1-5}$-Alkoxy)-carbonyl-, Cyan-oder $C_{1-4}$-Acylamidogruppe substituiert sein können, oder durch einen 5gliedrigen aromatischen Ring mit 1 bis 3 Stickstoffatomen oder einem Sauerstoff-oder Schwefelatom und ggf. 1-2 Stickstoffatomen, oder durch einen 6gliedrigen aromatischen Ring mit 1 bis 3 Stickstoffatomen, wobei diese heteroaromatischen Ringe ggf. 1 bis 3 $C_{1-3}$-Alkylgruppen tragen, substituiert sein können, bedeuten, oder $R^1$ und $R^2$ zusammen mit dem Stickstoffatom einen gesättigten heterocyclischen Ring mit 4 bis 12 Ringgliedern bilden, der durch eine Phenyl-und/oder eine Hydroxy-, eine $C_{1-4}$-Acyl-, eine ($C_{1-5}$-Alkoxy)-carbonyl-, eine Nitril-, eine N-Phenyl-N-($C_{1-4}$-Acyl)-amino-, eine N-Benzamidogruppe oder einen Benzimidazolin-2-on-1-yl-rest und/oder durch 1 bis 3 $C_{1-4}$-Alkylgruppen substituiert sein oder einen ankondensierten Benzolring tragen kann,

$R^3$, $R^4$ und $R^5$ unabhängig voneinander Wasserstoffatome oder $C_{1-3}$-Alkyl-gruppen bedeuten, wobei $R^4$ und $R^5$ gemeinsam mit der Doppelbindung auch einen Benzolring bedeuten können,

A einen $C_{2-8}$-Alkylenrest bedeutet, der einfach olefinisch oder acetylenisch ungesättigt sein kann und in dem ein Kohlenstoffatom durch ein Sauerstoff-, ein Schwefelatom oder eine Phenylengruppe ersetzt sein kann,

B eine der Gruppen $B^1$ bis $B^6$ bedeutet:

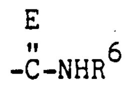,
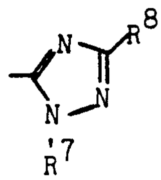,
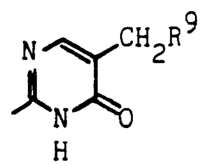

$B^1$ $B^2$ $B^3$

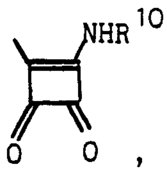,
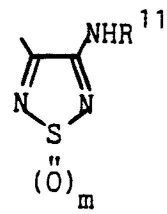
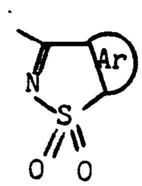

$B^4$ $B^5$ $B^6$

wobei

E S, $CH-NO_2$ oder N-CN bedeutet,

$R^6$ ein Wasserstoffatom oder einen $C_{1-3}$-Alkylrest, der durch einen 5gliedrigen aromatischen Ring mit 1 bis 3 Stickstoffatomen oder einem Sauerstoff- oder Schwefelatom und ggf. 1-2 Stickstoffatomen oder einen 6gliedrigen aromatischen Ring mit 1 bis 3 Stickstoffatomen, wobei diese heteroaromatischen Ringe ggf. 1 bis 3 $C_{1-3}$-Alkylgruppen tragen, oder durch eine Hydroxy-oder $C_{1-4}$ -Alkoxygruppe substituiert sein kann, bedeutet,

$R^7$ ein Wasserstoffatom oder einen $C_{1-3}$-Alkylrest bedeutet,

$R^8$ eine Aminogruppe oder den Rest $CH_2OR^{12}$ bedeutet, wobei

$R^{12}$ für ein Wasserstoffatom, einen $C_{1-3}$-Alkylrest, eine $C_{1-6}$-Acylgruppe, die Benzoylgruppe, den Tetrahydrofuran-2-yl oder Tetrahydropyran-2-yl-rest steht,

$R^9$ einen $C_{1-6}$-Alkylrest, eine Phenylgruppe, die durch je 1 oder 2 $C_{1-3}$--Alkyl-, $C_{1-3}$-Alkoxygruppen oder Chlor-oder Bromatome oder durch einen Methylendioxorest substituiert sein kann, oder einen 5gliedrigen aromatischen Ring mit 1 bis 3 Stickstoffatomen oder einem Sauerstoff-oder Schwefelatom und ggf. 1-2 Stickstoffatomen oder einen 6gliedrigen aromatischen Ring mit 1 bis 3 Stickstoffatomen, wobei diese heteroaromatischen Ringe ggf. 1 bis 3 $C_{1-3}$-Alkylgruppen tragen, bedeutet,

$R^{10}$ und $R^{11}$ Wasserstoffatome oder $C_{1-3}$-Alkylreste, die durch eine Phenylgruppe, die ihrerseits mit je 1 oder 2 $C_{1-3}$-Alkyl-, C $_{1-3}$-Alkoxygruppen oder Chlor- oder Bromatomen substituiert sein kann, oder durch einen 5gliedrigen aromatischen Ring mit 1 bis 3 Stickstoffatomen oder einem Sauerstoff-oder Schwefelatom und ggf. 1-2 Stickstoffatomen oder einem 6gliedrigen aromatischen Ring mit 1 bis 3 Stickstoffatomen, wobei diese heteroaromatischen Ringe ggf. 1 bis 3 $C_{1-3}$-Alkylgruppen tragen, oder durch eine Hydroxy-oder $C_{1-3}$ -Alkoxygruppe substituiert sein können, bedeuten,

m die Zahl 0, 1 oder 2 bedeutet und

Ar einen ankondensierten Benzol-oder Thiophenring darstellt, sowie deren physiologisch verträglichen Salze.

Für die einzelnen Reste können folgende bevorzugten Bedeutungen genannt werden:

$R^1$ und $R^2$ unabhängig voneinander ein Wasserstoffatom oder einen C1-12-Alkyl-oder $C_3$-$C_{12}$-Cycloalkylrest, die durch einen Phenylrest substituiert sein können, wobei nicht beide Reste $R^1$ und $R^2$ gleichzeitig Wasserstoffatome bedeuten, oder

$R^1$ und $R^2$ zusammen mit dem Stickstoffatom einen gesättigten Ring mit 5 bis 10 Ringgliedern, der durch eine Phenylgruppe substituiert sein oder einen ankondensierten Benzolring enthalten kann,

$R^3$, $R^4$ und $R^5$ Wasserstoffatome oder Methylgruppen, wobei $R^4$ und $R^5$ gemeinsam mit der Doppelbindung einen Benzolring bilden können,

A einen $C_{3-6}$-Alkylenrest, der gegebenenfalls anstelle eines C-Atoms ein Schwefelatom als Kettenglied enthält,

E die Gruppe N-CN oder $CH-NO_2$,

$R^6$ einen $C_{1-3}$-Alkylrest, der gegebenenfalls durch eine Hydroxylgruppe substituiert ist,

$R^7$ ein Wasserstoffatom oder die Methylgruppe,

$R^8$ die Aminogruppe oder die Hydroxymethylgruppe,

$R^9$ eine Phenylgruppe, die durch 1 oder 2 $C_{1-3}$-Alkoxygruppen substituiert ist oder eine Pyridylgruppe, die durch 1 - 2 $C_{1-3}$-Alkylgruppen substituiert sein kann,

$R^{10}$ ein Wasserstoffatom oder eine Methylgruppe,

$R^{11}$ ein Wasserstoffatom oder eine $C_{1-3}$-Alkylgruppe, die durch einen 5-oder 6gliedrigen heteroaromatischen Ring mit 1 bis 3 Stickstoffatomen substituiert sein kann, wobei dieser seinerseits ggf. durch 1 bis 3 $C_{1-3}$-Alkylgruppen substituiert ist,

m die Zahl 0 oder 1,

Ar einen ankondensierten Benzolring.

Bevorzugt bedeutet B eine der Gruppen $B^1$, $B^2$, $B^5$ oder $B^6$.

Besonders bevorzugte Bedeutungen der Reste sind:

$R^1$ eine $C_{1-12}$-Alkylgruppe und eine Benzylgruppe, oder $R^1$ und $R^2$ bilden zusammen mit dem Stickstoffatom einen 6-bis 7gliedrigen Ring, der durch eine Phenylgruppe substituiert oder benzokondensiert sein kann,

$R^2$ ein Wasserstoffatom und eine $C_{1-4}$-Alkylgruppe,

$R^3$, $R^4$ und $R^5$ Wasserstoffatome oder Methylgruppen,

A eine $C_{4-5}$-Alkylengruppe,

E die Gruppe $CH-NO_2$,

$R^6$ die Methylgruppe,

$R^7$ die Methylgruppe,

$R^8$ die Aminogruppe,

$R^9$ einen Pyridylrest, der durch 1 -2 Methylgruppen substituiert sein kann

$R^{10}$ ein Wasserstoffatom oder die Methylgruppe,

$R^{11}$ ein Wasserstoffatom, eine Methylgruppe oder eine Pyridylmethylgruppe,

m die Zahl 1,

B bedeutet besonders bevorzugt eine der Gruppen $B^1$ oder $B^2$. Eine weitere besonders bevorzugte Bedeutung von B stellt die Gruppe $B^5$ dar.

Die Erfindung schließt die Salze der Verbindungen der Formel I mit physiologisch annehmbaren Salzen ein. Als Säuren kommen beispielsweise Phosphorsäure, Essigsäure, Propionsäure, Malonsäure, Furansäure, Maleinsäure, Zitronensäure, Weinsäure oder Milchsäure in Betracht. Säuren wie HCl oder HBr, die das Pyrrolringsystem angreifen, sind weniger geeignet.

Weitere geeignete Säuren können aus Fortschritte der Arzneimittelforschung, Band 10, Seiten 224 bis 225, Birkhäuser Verlag, Basel und Stuttgart, 1966, oder dem Journal of Pharmaceuticals Sciences, Volume 66, Seiten 1 bis 5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Pyrrol-Derivate der allgemeinen Formel (I) durch Auflösen der freien Basen der allgemeinen Formel (I) in einer wäßrigen Säurelösung hergestellt werden.

Die Verbindungen der Formel I können in verschiedenen tautomeren Formen vorliegen; die Erfindung umfaßt alle tautomeren Formen. Bei Verbindungen, die in optischen Isomeren auftreten können, umfaßt die Erfindung alle optischen Isomeren.

Bei der nachfolgenden Beschreibung der Verfahren zur Herstellung von Verbindungen der Formel I oder von Zwischenprodukten der Formeln II bis XX haben die Reste $R^1$ bis $R^{12}$, A, B, E, m und Ar, wenn nichts anderes angegeben ist, die für Formel I angegebenen Bedeutungen.

Die erfindungsgemäßen Verbindungen der Formel I werden hergestellt, indem man ein Amin der Formel II

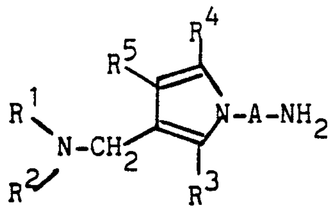 II,

mit Verbindungen B'Y, wobei Y eine geeignete Austrittsgruppe wie z.B. ein Halogenatom, wie Chlor oder Brom, eine $C_{1-3}$-Alkoxy-, eine $C_{1-3}$-Alkylthio-, eine $C_{1-3}$-Alkylsulfoxyl-, eine $C_{1-3}$-Alkylsulfonyl-, eine Phenoxy-oder Phenylthiogruppe bedeutet, und B' entweder die Reste $B^1$ -$B^6$ oder die unten beschriebenen Vorstufen $B'^1$ -$B'^6$ der Reste $B^1$ bis $B^6$ bedeutet, wobei die durch Umsetzung der Vorstufen $B'^1$ bis $B'^6$ erhaltenen Zwischenstufen in einem Folgeschritt in die erfindungsgemäßen Verbindungen übergeführt werden.

Im folgenden wird die Herstellung der einzelnen Verbindungsklassen näher erläutert.

Die Verbindungen der Formel I, in der B für die Gruppe $B^1$ steht, können durch die Umsetzung der Amine der Formel II mit Verbindungen der Formel III

$Y^1-\overset{E}{\overset{\|}{C}}-Z^1$ III,

in der $Z^1$ entweder für die Gruppe $NHR^6$ steht oder die Gruppe $Y^{1'}$ bedeutet, wobei $Y^1$ und $Y^{1'}$ unabhängig voneinander $C_{1-3}$-Alkoxy-, $C_{1-3}$-Alkylthio-, $C_{1-3}$-Alkylsulfoxyl-, $C_{1-3}$-Alkylsulfonyl-, Phenoxy- oder Phenylthioreste bedeuten, erhalten werden, wobei gegebenenfalls erhaltene Zwischenstufen der Formel IV

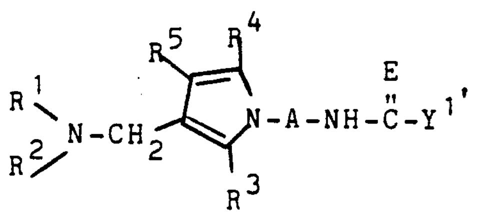 IV,

in der $Y^{1'}$ die oben angegebenen Bedeutungen besitzt, durch Umsetzung mit Aminen $R^6NH_2$ in die erfindungsgemäßen Verbindungen umgesetzt werden.

Verbindungen der Formel I, in der B den Rest $-\overset{S}{\overset{\|}{C}}-NHR^6$ bedeutet, können auch durch Umsetzung der Amine der Formel II mit Isothiocyanaten der Formel $R^6N=C=S$ erhalten werden.

Verbindungen der Formel I, in der B die Gruppe $B^2$ bedeutet, können durch Umsetzung der Amine der Formel II mit Verbindungen der Formel V

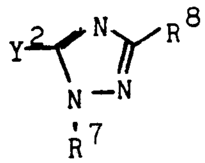 V,

in der $Y^2$ ein Halogenatom, eine $C_{1-3}$-Alkylthio-, eine $C_{1-3}$-Alkylsulfoxyl-oder $C_{1-3}$-Alkylsulfonylgruppe bedeutet und der Rest $R^8$, falls er eine Aminogruppe oder die Hydroxymethylgruppe bedeutet, ggf. durch eine geeignete Schutzgruppe geschützt ist, erhalten werden. Ggf. vorhandene Schutzgruppen im Rest $R^8$ werden nach der Umsetzung der Amine II mit den Verbindungen V in üblicher Weise abgespalten.

Durch Umsetzung der Amine der Formel II mit Verbindungen der Formel VI

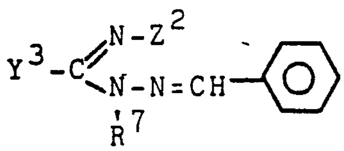 VI,

worin $Y^3$ eine $C_{1-3}$-Alkoxy-oder $C_{1-3}$-Alkylthiogruppe oder ein Halogenatom bedeutet und $Z^2$ die Nitrilgruppe oder die Gruppe $-\overset{O}{\overset{\|}{C}}-CH_2-OR^{12'}$ bedeutet, wobei $R^{12'}$ die Bedeutung von $R^{12}$ besitzt mit der Ausnahme, daß $R^{12'}$ kein Wasserstoffatom sein soll, zu den Zwischenstufen der Formel VII

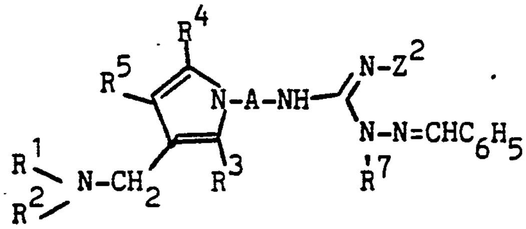 VII,

und anschließende Hydrolyse der Benzaldehydhydrazongruppe unter gleichzeitiger Cyclisierung mit organischen Säuren, wie z.B. Essigsäure oder Weinsäure, werden ebenfalls die erfindungsgemäßen Verbindungen der Formel I, $B=B^2$ erhalten. Falls gewünscht, können Verbindungen der Formel I, $B=B^2$, in der $R^8$ die Gruppe $CH_2OR^{12''}$ bedeutet, worin $R^{12''}$ eine $C_{1-6}$-Acylgruppe, die Benzoylgruppe, den Tetrahydrofuran-2-yl-oder Tetrahydropyran-2-yl-rest bedeutet, durch alkalische Hydrolyse bzw. saure Acetalspaltung in die Verbindungen der Formel I, $B=B^2$, worin $R^8$ die Hydroxymethylgruppe bedeutet, umgewandelt werden.

Verbindungen der Formel I, $B=B^2$, in der $R^7$ ein Wasserstoffatom bedeutet, können auch durch Umsetzung der Verbindungen der Formel IV'

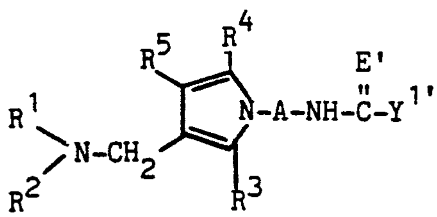 IV',

in der $Y^{1'}$ die oben angegebene Bedeutung besitzt und E' die Gruppe N-CN oder $N-\overset{O}{\overset{\|}{C}}-CH_2OR^{12}$ bedeutet, mit Hydrazin erhalten werden.

Verbindungen der Formel I, in der B den Rest $B^3$ bedeutet, können durch Umsetzung der Amine der Formel II mit den Verbindungen der Formel VIII

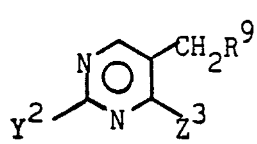 VIII,

in der $Y^2$ und $R^9$ die oben angegebenen Bedeutungen besitzen und $Z^3$ eine Benzyloxygruppe bedeutet, und anschließende Entbenzylierung der Benzyloxygruppe $Z^3$ erhalten werden.

Verbindungen der Formel I, in der B für den Rest $B^4$ steht, können durch Umsetzung der Amine der Formel II mit Verbindungen der Formel IX

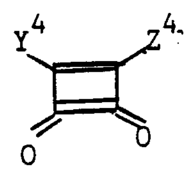

IX,

wobei $Y^4$ ein Halogenatom, eine $C_{1-3}$-Alkoxy-oder $C_{1-3}$-Alkylthiogruppe und $Z^4$ die Gruppe $NHR^{10}$ oder den Rest $Y^4$ bedeutet, und ggf. Umsetzung der erhaltenen Zwischenprodukte der Formel X

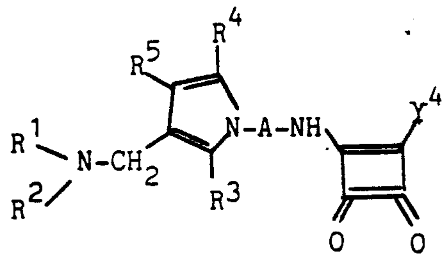

X,

mit Aminen $R^{10}NH_2$ erhalten werden.

Verbindungen der Formel I, in der B die Gruppe $B^5$ bedeutet, können durch Umsetzung der Amine der Formel II mit Verbindungen der Formel XI

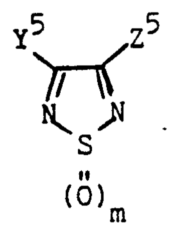

XI,

in der $Y^5$ ein Halogenatom, eine $C_{1-3}$-Alkoxygruppe, eine ($C_{1-3}$-Alkoxy)-ethoxygruppe oder eine $C_{1-3}$-Alkylthiogruppe bedeutet und $Z^5$ den Rest $NHR^{11}$ oder $Y^5$ und m die Zahlen 0, 1 oder 2, und Umsetzung der ggf. erhaltenen Zwischenprodukte der Formel XII

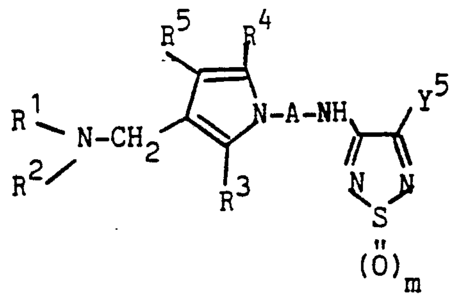

XII,

mit Aminen $R^{11}NH_2$ erhalten werden.

Die Verbindungen der allgemeinen Formel I, in der B den Rest $B^6$ bedeutet, können durch Umsetzung der Amine der Formel II mit Verbindungen der Formel XIII

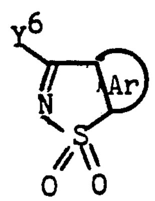

XIII,

in der $Y^6$ ein Halogenatom, eine $C_{1-3}$-Alkoxygruppe oder eine $C_{1-3}$ -Alkylthiogruppe bedeutet, erhalten werden.

Bei der Herstellung der Verbindungen der Formel I, in der einer der Reste $R^1$ oder $R^2$ ein Wasserstoffatom bedeutet, kann die Einführung einer Schutzgruppe am Stickstoffatom des Restes $R^1R^2N$-in den Aminen der Formel II von Vorteil sein. Geeignete Schutzgruppen sind beispielsweise die Formyl-oder Benzyloxycarbonylgruppe. Diese Schutzgruppen werden in üblicher Weise nach erfolgter Umsetzung wieder abgespalten.

Die Ausgangsverbindungen der Formeln III, V, VI, VIII, IX, XI und XIII sind bekannt, bzw. lassen sich analog literaturbekannten Verfahren herstellen. Ihre Herstellung, sowie die Reaktionsbedingungen für die Umsetzung mit Aminen sind beispielsweise in den folgenden Patentanmeldungen bzw. Patentschriften beschrieben:

$B = B^1$: DE-OS 27 34 070, EP-A 1699, EP-A 14 064;

$B = B^2$: DE-OS 29 17 026, EP-A 16 565;

$B = B^3$: EP-A 58 055, US 42 16 318;

$B = B^4$: DE-OS 32 18 584;

$B = B^5$: DE-OS 30 33 169, DE-OS 3 311 128, EP-A 40 696;

$B = B^6$: EP-A 81 955.

Die Umsetzungen der Amine der Formel II zu den erfindungsgemäßen Verbindungen der Formel I wurden in Analogie zu den in den oben erwähnten Patentanmeldungen und Patentschriften durchgeführt.

Die Zwischenstufen der Formel II können durch eine reduktive Aminierung von Verbindungen der Formel XIV

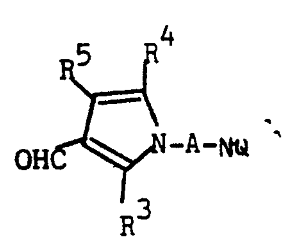

XIV,

in der die Gruppe NQ eine durch eine geeignete Schutzgruppe geschützte Aminogruppe, wie z.B. die Formylamino, Phthalimido, Benzyloxycarbonylamino-, Pyridin-3-ylmethyloxycarbonylamino-, (6-Methylpyridin-3-yl)-methyloxycarbonylamino-oder t-Butoxycarbonylaminogruppe bedeutet, mit Aminen der Formel $R^1R^2NH$ zu den Verbindungen der Formel XV

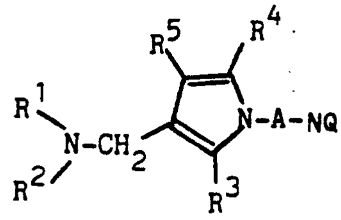

XV,

in der die Gruppe NQ die obige Bedeutung besitzt, und anschließende Abspaltung der Schutzgruppe erhalten werden. Die Zwischenprodukte der Formeln XV und II besitzen ebenfalls Histamin-$H_2$-Rezeptoren-blockierende Wirkungen.

Die reduktive Aminierung kann mit Wasserstoff in Gegenwart eines metallischen Katalysators wie Nickel-, Kobalt-oder Platinkatalysatoren oder mit metallorganischen Hydriden wie $NaBH_4$ oder $NaBH_3CN$, oder mit Ameisensäure durchgeführt werden.

Verbindungen der Formel XV, in der entweder die Reste $R^3$ und $R^4$ $C_{1-3}$-Alkylgruppen bedeuten oder die Reste $R^4$ und $R^5$ zusammen mit der Doppelbindung einen Benzolring bilden, können auch durch eine Mannichreaktion der Verbindungen der Formel XVI

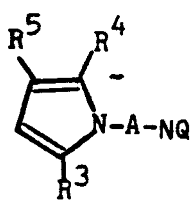

XVI,

in der $R^3$ und $R^4$ $C_{1-3}$-Alkylgruppen bedeuten oder $R^4$ und $R^5$ zusammen mit der Doppelbindung einen Benzolring bilden, mit Formaldehyd und Aminen der Formel $R^1R^2NH$ erhalten werden. Die Mannichreaktion kann in Gegenwart einer organischen Carbonsäure durchgeführt werden.

Verbindungen der Formel XV, in der die Reste $R^4$ und $R^5$ gemeinsam mit der Doppelbindung einen Benzolring bilden, können auch durch die Umsetzung einer Verbindung der Formel XVII

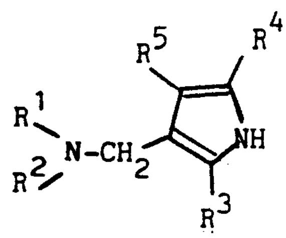

XVII,

in der $R^4$ und $R^5$ zusammen mit der Doppelbindung einen Benzolring bilden, mit Verbindungen der Formel XVIII

$Y^7$-A-NQ XVIII,

in der $Y^7$ eine Austrittsgruppe, z.B. ein Halogenatom wie Chlor, Brom oder Iod, oder eine Sulfonestergruppe wie die Methansulfonyloxy-, Trifluormethansulfonyloxy-oder p-Toluolsulfonyloxygruppe darstellt, in Gegenwart einer Base umsetzt. Als Basen kommen beispielsweise $K_2CO_3$, $NaOCH_3$, NaH oder Butyllithium in Betracht.

Verbindungen der Formel XIV, in der $R^3$ bis $R^5$ Wasserstoffatome oder $C_{1-3}$-Alkylgruppen darstellen, wobei mindestens einer der Reste $R^3$ oder $R^4$ ein Wasserstoffatom bedeutet, können durch Umsetzung der Verbindungen der Formel XIX

$NH_2$-A-NQ XIX

mit Verbindungen der Formel XX

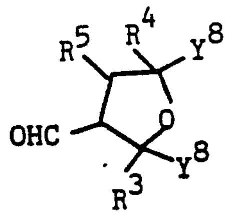

XX,

in der $Y^8$ ein Halogenatom, wie Chlor-oder Brom, eine $C_{1-6}$-Alkanoyloxygruppe wie Acetoxy, Propionyloxy oder eine $C_{1-4}$-Alkoxygruppe, wie Methoxy, Ethoxy oder n-Butoxy, bedeutet, erhalten werden. Die Reaktion kann in niederen Carbonsäuren wie z.B. Essigsäure oder Propionsäure oder in organischen Lösungsmitteln wie z.B. Kohlenwasserstoffen wie Toluol oder Cyclohexan in Gegenwart von 0.01-120 Mol% eines sauren Katalysators wie Toluolsulfonsäure, Schwefelsäure oder eines sauren Ionenaustauschers durchgeführt werden.

$Y^8$ bedeutet bevorzugt eine $C_{1-4}$-Alkoxygruppe, insbesondere die Methoxygruppe.

Verbindungen der Formel XIV, in denen entweder $R^3$ und $R^4$ $C_{1-3}$-Alkylgruppen bedeuten, oder $R^4$ und $R^5$ gemeinsam mit der Doppelbindung einen Benzolring bilden, können durch Umsetzung der Verbindungen der Formel XVI, in der entweder $R^3$ und $R^4$ $C_{1-3}$-Alkylgruppen bedeuten oder $R^4$ und $R^5$ gemeinsam mit der Doppelbindung einen Benzolring bilden, durch Umsetzung mit tertiären Formamiden, wie N,N-Dimethylformamid oder N-Phenyl-N-methylformamid in Gegenwart von anorganischen oder organischen Säurechloriden wie $POCl_3$, $SOCl_2$ oder Oxalylchlorid synthetisiert werden.

Die erfindungsgemäßen Verbindungen besitzen Histamin-$H_2$-Rezeptoren-blokkierende Wirkung und hemmen die Magensäuresekretion. Sie eignen sich z. B. zur Therapie des Magen-oder Duodenalulkus, sowie von gastrointestinalen Erkrankungen, deren Heilung durch eine Erniedrigung der Säuresekretion gefördert wird.

Histamin-$H_2$-Rezeptor-blockierende Verbindungen des allgemeinen Typs D

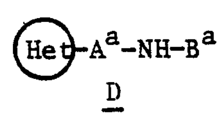

D

worin Het verschiedene 5-oder 6gliedrige aromatische Heterocyclen, die mit einer Gruppe $RR'NCH_2$-substituiert sein können, und $A^a$ eine Alkylenkette bedeutet, in der eine Methylengruppe durch ein Schwefel-oder Sauerstoffatom ersetzt sein kann und $B^a$ beispielsweise die Bedeutung des Restes B der Verbindungen der allgemeinen Formel I besitzt, wurden beispielsweise in der DE-OS 23 44 779, DE-OS 2 917 026, EP-A 40 696, EP-55 179 und GB 20 84 581 beschrieben. Bedeutete Het in D einen 5gliedrigen aromatischen Heterocyclus mit einem Heteroatom wie O, S oder N im Ring, so erfolgte die Verknüpfung der Seitenkette $A^a$-NH-$B^a$ in den bevorzugten Verbindungen ausschließlich in der 2-Position des Heterocyclus. Die Verknüpfung des Restes $A^a$-NH-$B^a$ mit der 1-Position eines Pyrrolrestes wurde von Morishita in der JA-OS 56 068 666 beschrieben. Die Verbindungen der allgemeinen Formel E,

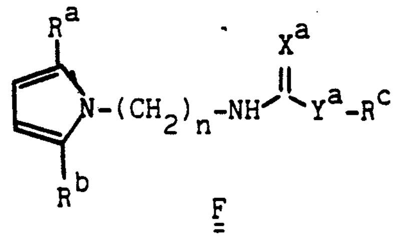

E

worin $R^a$, $R^b$ und $R^c$ niedere Alkylgruppen, n eine ganze Zahl von 2 -5, $X^a$ ein Schwefelatom oder die Gruppe N-CN und $Y^a$ ein Schwefelatom oder die Gruppe NH bedeutet, sind zur Therapie und zur Vorbeugung von Magen-/Darm-Ulcus geeignet. Aus der JA-OS 56 068 666 wurde auf Grund ihrer gemeinsamen Strukturmerkmale mit Cimetidin die Verbindung des Beispiels 12 (E: $R^a = R^b = R^c = CH_3$, n = 4, $X^a$ = NCN, $Y^a$ = NH) ausgewählt und auf ihre Histamin-$H_2$-Rezeptoren-blockierende Wirkung untersucht. Diese Verbindung ist wesentlich - schwächer wirksam als Cimetidin. Es. war daher nicht zu erwarten, daß die erfindungsgemäßen Verbindungen gute Histamin-$H_2$-rezeptorblockierende Eigenschaften besitzen sollten. Überraschenderweise übertreffen die erfindungsgemäßen Verbindungen in ihrer Wirksamkeit sowohl die Verbindung 12 der JA-OS 56 068 666 als auch Cimetidin sehr deutlich.

Die erfindungsgemäßen Verbindungen der Formel I und ihre physiologisch verträglichen Säureadditionssalze weisen wertvolle pharmakologische Eigenschaften auf und können als Wirkstoffe zur Behandlung von Krankheitszuständen, die mit einer gesteigerten Magensäureproduktion einhergehen, verwendet werden. Die Erfindung betrifft dementsprechend auch therapeutische Mittel, gekennzeichnet durch einen Gehalt an einer Verbindung der Formel I oder deren pharmakologisch verträglichem Säureadditionssalz als Wirkstoff neben üblichen Träger-und Verdünnungsmitteln.

Therapeutische Mittel mit üblichen Trägerstoffen oder Verdünnungsmitteln und den üblicherweise verwendeten technischen Hilfsstoffen können entsprechend der gewünschten Applikationsart und mit einer zur Anwendung geeigneten Dosiereinheit in an sich üblicher Weise hergestellt werden.

Die neuen Verbindungen können in den üblichen galenischen Applikationsformen, fest oder flüssig, angewendet werden. Diese werden in üblicher Weise hergestellt. Die Wirkstoffe können dabei mit den gebräuchlichen galenischen Hiflsmitteln, bei Tabletten beispielsweise mit inerten Bindeund Verdünnungsmitteln wie Stärke, Gelatine, Gummiarabicum, Dextrose, Saccharose, Lactose, Zucker, Sorbit, Mannit, Polyvinylpyrrolidon, Polyvinylalkohol, Calciumcarbonat, Calciumphosphat, Michzucker; Sprengmitteln wie Maisstärke oder Alginsäure; Gleitmitteln wie Magnesiumstearat oder Talkum und/oder Mitteln zur Erzielung eines Depoteffektes, wie Carboxypolymethylen, Carboxymethylcellulose, Celluloseacetatphthalat oder Polyvinylacetat, bei flüssigen Applikationsformen mit wäßrigen oder nicht wäßrigen Trägern, Netzmitteln, Dipsergiermitteln, Emulgatoren, Geschmackstoffe und/oder Konservierungsmitteln verarbeitet werden (vgl. z.B. Hagers Handbuch der Pharmazeut. Praxis, 4. Aufl., Springer-Verl. Berlin -Heidelberg -New York, 1967-1980. Bd. VII A: Arzneiformen (B: Hilfs stoffe); H. Sucker et al., Pharmazeutische Technologie, Thieme-Verlag, Stuttgart 1978). Die so erhaltenen Präparate enthalten den Wirkstoff normalerweise in einer Menge von 0,001 bis 99 Gew.%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Granulate, Lösungen, Suspensionen oder Depotformen. Es kommen auch parenterale Zubereitungen, wie Injektionslösungen, in Betracht. Weiterhin seien als Zubereitungen beispielsweise auch Suppositorien genannt.

Die Histamin-$H_2$-Rezeptor-antagonistische Wirkung der Verbindungen wurde im Modell der Antagonisierung der durch Dimaprit, einer spezifischen Histamin-$H_2$-Agonisten, ausgelösten Blutdrucksenkung an der narkotisierten Ratte bei intravenöser Gabe nachgewiesen.Die Dosierung bei der therapeutischen Anwendung liegt im Bereich von 20 bis 1500 mg/Patient und Tag, bevorzugt bei 100 bis 1000 mg. Sie kann in 1 bis 4 Dosen/Tag aufgeteilt werden.

Zum Nachweis der Histamin-$H_2$-Rezeptor-blockierenden Wirkung wird folgende Methode angewendet.

An narkotisierten (Urethan-Narkose: 1,78 g/kg i.p.) Ratten (Stamm: Sprague-Dawley, männlich, Gewicht: 210 -310 g) verursacht der spezifische $H_2$-Rezeptor-Agonist Dimaprit (S-[3-(N,N-dimethylamino)propyl]isothio urea) (Literatur siehe: PARSONS, M. E. et al., Agents and Actions 7, 31 -37 -(1977); FLYNN, Sh. B. et al., Brit. J. Pharmac. 61, 101 -107 (1977) nach i.v. Injektion von 3,16 mg/kg Blutdrucksenkungen um durchschnittlich 27 ± 0,7 mmHg bei Ausgangsdrucken von 100 ± 1, 1 mmHg (Anzahl der Tiere N = 70). $H_2$-Rezeptor-Antagonisten wie beispielsweise Cimetidin hemmen diese Dimaprit-Blutdrucksenkung dosisabhängig. Die Applikation der geprüften Substanzen erfolgt intravenös 5 min vor der Dimaprit-Injektion. Als ED 50 % werden die Dosen ermittelt, welche die Dimaprit-Blutdrucksenkung um 50 % hemmen.

Herstellungsbeispiele für Ausgangsverbindungen

Beispiele IA -IM

Herstellung von Zwischenprodukten der allgemeinen Formel XIV durch Umsetzung der Tetrahydrofurancarbaldehyde der Formel XX mit monogeschützten Diaminen der Formel XIX.

Beispiel IA

Eine Lösung von 276 g (1.72 Mol) 2,5-Dimethoxytetrahydrofuran-3-carbaldehyd und 200 g einer Mischung aus Mono-, Diformyldiaminobutan und Diaminobutan (s. u.) in 800 ml Essigsäure wurde 2 Stunden bei 80 °C gerührt. Nach Abziehen der Essigsäure am Rotationsverdampfer wurde der Rückstand mit 1 l gesättigter $NaHCO_3$-Lösung versetzt und mit $CH_2Cl_2$ extrahiert. Nach Trocknen über Natriumsulfat und Abziehen des Lösungsmittels wurde der Rückstand über Kieselgel mit $CH_2Cl_2/CH_3OH$ (95/5) chromatographiert. Man erhielt 88 g N-[4-(3-Formylpyrrol-1-yl)butyl]-formamid als Öl.

$^1$H-NMR ($d_6$-DMSO): $\delta$ = 1,0-2,0 (m, 4H), 3,1 - (m, 2H), 3,95 (t, 2H),

6,45 (m, 1H), 6,92 (m,1H), 7,63 (m, 1H), 8,00 (br.s, 1H), 9,65 (s, 1H).

Die Mischung aus Mono-, Diformyl-diaminobutan und Diaminobuten wurde durch Umsetzung von 388 g (4,4 Mol) Diaminobutan und 345 g (5,75 Mol) Ameisensäuremethylester in Gegenwart von 36 g (50 mMol) 30prozentiger Natriummethylatlösung bei 50 °C erhalten. Nach Stehen über Nacht wurde auskristallisiertes Diformyldiaminobutan abfiltriert und das Filtrat am Rotationsverdampfer eingeengt. Die erhaltene Mischung aus Mono- und Diformyldiaminobutan und Diaminobutan wurde direkt in die obige Reaktion eingesetzt.

Analog Beispiel IA wurden die Verbindungen der Beispiele IB -IJ hergestellt.

Beispiele IB -IJ

Verbindungen der Formel XIV, $R^3=R^4=R^5=H$, NQ=NHCHO

| Beispiel | -A- |
|---|---|
| IB | $-(CH_2)_2-$ |
| IC | $-(CH_2)_3-$ |
| ID | $-(CH_2)_5-$ |
| IE | $-(CH_2)_6-$ |
| IF | $-(CH_2)_8-$ |
| IG | $-(CH_2)_2-S-(CH_2)_2-$ |
| IH | $-(CH_2)_2-O-(CH_2)_2-$ |
| IJ | m-Xylylen |

Beispiel IK

Durch Umsetzung von 100 g (0.57 Mol) einer Mischung aus 2.5-Dimethoxy-2-methyltetrahydrofuran-4-carbaldehyd und 2.5-Dimethoxy-2-methyltetrahydrofuran-3-carbaldehyd - (~4 : 1) mit 66,7 g einer Mischung aus Diformyl-, Monoformyl-1,4-diaminobutan und 1,4-Diaminobutan analog Beispiel IA wurden nach Säulenchromatographie 26 g einer Mischung aus 4 Teilen N[4-(4-Formyl-2-methyl-pyrrol-1-yl)butyl]-formamid und 1 Teil N-[4-(3-Formyl-2-methyl-pyrrol-1-yl)butyl]formamid erhalten. Durch eine zweite Säulenchromatographie konnte der Gehalt an N-[-(4-Formyl-2-methyl-pyrrol-1-yl)-butyl]-formamid auf≥90 % erhöht werden.

$^1$H-NMR ($d_6$-DMSO): $\delta$ = 1,1-2,0 (m, 4H), 2,20 - (s, 3H), 3,15 (m, 2H),

3,95 (t, 2H), 6,25(m, 1H), 7,55 (m, 1H),

8,05 (br.s, 1H), 9,60 (s, 1H).

Beispiel IL

Analog Beispiel IK wurde N-[5-(4-Formyl-2-methylpyrrol-1-yl)pentyl]-formamid erhalten.

Beispiel IM

Durch Umsetzung analog Beispiel IA von 65 g (0,29 Mol) Monobenzyloxycarbonyldiaminobutan mit 51,6 g (0,32 Mol) 2,5-Dimethoxytetrahydrofuran-3-carbaldehyd in 500 ml Essigsäure bei 80° wurden nach Säulenchromatographie und Umkristallisation aus DMF/$H_2O$ 51 g N-[4-(3-Formyl-pyrrol-1-yl)-butyl]-O-benzylcarbamat vom Fp. 48 -49 °C erhalten.

Beispiele IIA -VE: Verbindungen der allgemeinen Formel XV

Beispiel IIA

31,9 g (0,16 Mol) der Verbindung aus Beispiel IA wurde mit 14,7 g (0,17 Mol) Piperidin und 3 g Platin/Kohle in 70 ml Ethanol bei Raumtemperatur bis zur Beendigung der Wasserstoffaufnahme (10 Stunden) hydriert. Nach Filtration und Abziehen des Lösungsmittels wurde das Rohprodukt säulenchromatographisch gereinigt (Kieselgel, $CH_2Cl_2$/$CH_3OH$ 4 : 1). Man erhielt 10,5 g N-[4-(3-Piperidinomethyl-pyrrol-1-yl)butyl]-formamid als Öl.

$^1$H-NMR ($d_6$-DMSO): $\delta$ = 1,0-2,0 (m, 10H), 2,1-2,6 (m, 4H), 3,1 (m, 2H),

3,2 (s, 2H), 3,8 (t, 2H), 4,5 (br.s, 1H),

5,9 (m, 1H), 6,6 (m, 2H), 8,0 (br.s, 1H).

Beispiele IIB -IIBC

Analog Beispiel IIA wurden die Verbindungen der Beispiele IIB bis IIV und IIAA bis IIAD und IIBA bis IIBC erhalten.

Beispiele IIB - IIV: Verbindungen der Formel XV, $R^3=R^4=R^5=H$, NQ=-NH-CHO

| Beispiel | $R^1$ | $R^2$ | -A- |
|---|---|---|---|
| IIB | $-(CH_2)_5-$ | | $-(CH_2)_2-$ |
| IIC | $-(CH_2)_5-$ | | $-(CH_2)_3-$ |
| IID | $-(CH_2)_5-$ | | $-(CH_2)_5-$ |
| IIE | $-(CH_2)_5-$ | | $-(CH_2)_6-$ |
| IIF | $-(CH_2)_5-$ | | $-(CH_2)_8-$ |
| IIG | $-(CH_2)_5-$ | | $-(CH_2)_2-S-(CH_2)_2-$ |
| IIH | $-(CH_2)_5-$ | | $-(CH_2)_2-O-(CH_2)_2-$ |
| IIJ | $C_6H_5CH_2-$ | $CH_3$ | $-(CH_2)_5-$ |
| IIK | $n-C_4H_9-$ | $n-C_4H_9-$ | $-(CH_2)_4-$ |
| IIL | $C_6H_5CH_2-$ | $CH_3-$ | $-(CH_2)_4-$ |
| IIM | $-(CH_2)_4-$ | | $-(CH_2)_4-$ |
| IIN | $-(CH_2)_6-$ | | $-(CH_2)_4-$ |
| IIO | $-(CH_2)_2-CH(C_6H_5)-(CH_2)_2-$ | | $-(CH_2)_4-$ |
| IIP | $-CH_2-(o-C_6H_4)-CH_2-CH_2-$ | | $-(CH_2)_4-$ |
| IIQ | $CH_3-$ | $CH_3-$ | $-(CH_2)_5-$ |
| IIR | $-(CH_2)_2-CH(C_6H_5)-(CH_2)_2-$ | | $-(CH_2)_5-$ |
| IIS | $-CH_2-CH(C_6H_5)-(CH_2)_2-$ | | $-(CH_2)_5-$ |
| IIT | $-CH_2-(o-C_6H_4)-CH_2-CH_2-$ | | $-(CH_2)_5-$ |
| IIU | $C_6H_5CH_2-$ | $CH_3-$ | m-Xylylen |
| IIV | $-(CH_2)_5-$ | | m-Xylylen |

Beispiele IIAA -IIAD

Verbindungen der Formel XV, $R^3=R^4=R^5=H$, NQ=-NHCHO

| Beispiel | $R^1$ | $R^2$ | -A- |
|---|---|---|---|
| IIAA | $(CH_3)_2CH-CH_2-$ | H | $-(CH_2)_4-$ |
| IIAB | $c-C_6H_{11}-$ | H | $-(CH_2)_4-$ |
| IIAC | $C_6H_5CH_2-$ | H | $-(CH_2)_4-$ |
| IIAD | $n-C_{12}H_{25}-$ | H | $-(CH_2)_5-$ |

Beispiele IIBA -IIBC

Verbindungen der Formel XV, $R^3=R^5=H$, $R^4=CH_3$, NQ=-NHCHO

| Beispiel | $R^1$ | $R^2$ | -A- |
|---|---|---|---|
| IIBA | $-(CH_2)_5-$ | | $-(CH_2)_4-$ |
| IIBB | $C_6H_5-CH_2-$ | $CH_3-$ | $-(CH_2)_5-$ |
| IIBC | $-(CH_2)_5-$ | | $-(CH_2)_5-$ |

Beispiel IIIA

14 g (62 mMol) der Verbindung des Beispiels IG wurden mit 6,3 g (74 mMol) Piperidin, 8,9 g (74 mMol) Essigsäure und 2,3 g (38 mMol) $NaBH_3CN$ in 100 ml Methanol über Nacht bei Raumtemperatur gerührt. Nach Abziehen des Lösungsmittels wurde der Rückstand in Wasser aufgenommen und mit NaOH alkalisch gestellt. Nach Extraktion mit Methylenchlorid wurde die organische Phase mit 1 N NaOH und $H_2O$ gewaschen, über $Na_2SO_4$ getrocknet und am Rotationsverdampfer eingeengt.

Nach chromatographischer Reinigung über Kieselgel ($CH_2Cl_2/CH_3OH$ 4 : 1) erhielt man 8.5 g N-{2-[2-(3-Piperidinomethyl-pyrrol-1-yl)-ethylthio]-ethyl}-formamid als Öl.

$^1$H-NMR ($CD_3OD$): $\delta$ = 1,2-1,8 (m, 6H), 2,3-2,7 (m, 6H), 2,85 (t, 2H),

3,3 (m, 2H), 3,4 (s, 2H), 4,05 (t, 2H), 6,05 (m, 1H),

6,70 (m, 2H), 8,0 (br.s, 1H).

Beispiel IIIB

Analog Beispiel IIIA wurde durch Umsetzung von 15 g (50 mMol) der Verbindung des Beispiels IM, 12 g (100 mMol) N-Benzyl-N-methylamin, 6 g - (100 mMol) Essigsäure und 3 g (50 mMol) $NaBH_3CN$ 16,5 g N-[4-[3-(N-Benzyl-N-methylaminomethyl)-pyrrol-1-yl]butyl]-O-benzylcarbamat erhalten (Öl).

$^1$H-NMR ($d_6$-DMSO): $\delta$ = 1,35 (m, 2H), 1,65 (m, 2H), 2,1 (s, 3H),

3,0 (q, 2H), 3,43 (s, 2H), 3,52 (s, 2H),

3,83 (t, 2H), 5,02 (s, 2H), 6,00 (s, 1H),

6,68 (s, 2H), 7,32 (m, 10H).

Die Verbindungen der Beispiele IIA bis IIV, IIAA bis IIAD und IIBA bis IIBC können auch analog Beispiel IIIA hergestellt werden.

Beispiele IVA -IVC

Verbindungen der Formel XV, $R^3 = R^4 = CH_3$, $R^5 = H$, -NQ = -NHCHO

Beispiel IVA

Eine Lösung aus 10,5 g (50 mMol) N[5-(2,5-Dimethyl-pyrrol-1-yl)-pentyl]-formamid, 4,3 g (50 mMol) Piperidin und 3 g (50 mMol) Essigsäure in 200 ml Ethanol wurde bei 0 °C mit 4 g (50 mMol) 37prozentiger Formaldehydlösung versetzt und 1 Stunde bei 0 °C und über Nacht bei Raumtemperatur gerührt. Nach Abziehen des Lösungsmittels wurde der Rückstand in Wasser aufgenommen, mit NaOH alkalisch gestellt und mit Methylenchlorid extrahiert. Nach Trocknen über $Na_2SO_4$ und Abziehen des Lösungsmittels wurde das Rohprodukt säulenchromatographisch gereinigt (Kieselgel, $CH_2Cl_2/CH_3OH$ 9 : 1). Man erhielt 12,4 g N-5-(2,5-Dimethyl-3-piperidinomethyl-pyrrol-1-yl)pentyl]-formamid als Öl.

$^1$H-NMR ($d_6$-DMSO): $\delta$ = 1,0-1,9 (m, 12H), 1,9-2,4 (m, 4H), 2,12 (s, 6H),

2,9-3,3 (m, 2H), 3,15 (s, 2H), 3,5-3,8 (m, 2H),

5,6 (s, 1H), 8,0 (br.s, 1H).

Beispiele IVB und IVC

Analog Beispiel IVA wurden durch Umsetzung von N[5-(2.5-Dimethylpyrrol-1-yl)pentyl]formamid mit Dimethylamin bzw. N-Benzyl-N-methylamin die Verbindungen der Beispiele IVB -IVC erhalten.

Verbindungen der Formel XV, $R^3$=$R^4$=$CH_3$, $R^5$=H, -NQ=-NHCHO

| Beispiel | $R^1$ | $R^2$ | -A- |
|---|---|---|---|
| IVB | $CH_3$- | $CH_3$- | $-(CH_2)_5-$ |
| IVC | $C_6H_5CH_2$- | $CH_3$- | $-(CH_2)_5-$ |

Beispiele VA -VE

Verbindungen der Formel XV,

$R^3$=H, $R^4$ + $R^5$= 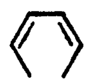 , -NQ= 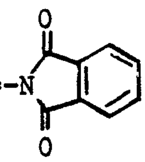 .

Beispiel VA

Zu einer Suspension von 4,8 g (0,2 Mol) NaH in 100 ml absolutem DMF wurde bei Raumtemperatur unter Stickstoff eine Lösung von 32,1 g (0,15 Mol) 3-(Piperidinomethyl)-indol in 150 ml absolutem DMF zugetropft. Nach 3Ominütigem Rühren wurde eine Lösung von 53,6 g (0,15 Mol) N-(6-Jodhexyl)phthalimid in 50 ml DMF zugegeben und die Mischung über Nacht bei Raumtemperatur gerührt. Nach Abziehen des DMF wurde mit Wasser versetzt und mit $CH_2Cl_2$ extrahiert. Nach Waschen der organischen Phase mit Wasser, Trocknen über $Na_2SO_4$ und Abziehen des Lösungsmittels am Rotationsverdampfer wurde der Rückstand über Kieselgel chromatographiert (Aceton/Methanol 99 : 1). Man erhielt 38,3 g N-{6-[3-(Piperidinomethyl)indol-1-yl]-hexyl}-phthalimid als Öl.

$^1$H-NMR ($d_6$-DMSO): $\delta$ = 1,0-2,0 (m, 14H), 2,6 - (m, 4H), 3,6 (t, 2H),

3,9 (s, 2H), 4,2 (t, 2H), 7,1-8,0 (m, 5H),

8,0 (s, 4H).

Beispiele VB -VE

Analog Beispiel VA wurden durch Umsetzung der entsprechenden 3-Aminomethylindole mit ω-Jodalkylphthalimiden die Verbindungen der Beispiele VB -VE erhalten.

Verbindungen der Formel XV $R^3$=H, $R^4$ + $R^5$ = 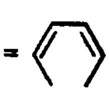 , -NQ= 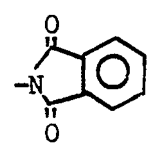

| Beispiel | $R^1$ | $R^2$ | -A- |
|---|---|---|---|
| VB | $-(CH_2)_5-$ | | $-(CH_2)_4-$ |
| VC | $-(CH_2)_5-$ | | $-(CH_2)_5-$ |
| VD | $CH_3$- | $CH_3$- | $-(CH_2)_5-$ |
| VE | $C_6H_5$-$CH_2$- | $CH_3$- | $-(CH_2)_5-$ |

Beispiele VI A -IX E Verbindungen der allgemeinen Formel II

Beispiel VI A

Eine Lösung von 2,0 g (7,6 mMol) der Verbindung aus Beispiel II A und 35 ml 1 n NaOH in 30 ml $CH_3OH$ wurde 10 Stunden bei 50° gerührt. Nach Einengen des Reaktionsansatzes wurde mit $CH_2Cl_2$ extrahiert, der Extrakt über $Na_2SO_4$ getrocknet und vom Lösungsmittel befreit. Man erhielt 1,6 g 4-[3-(Piperidinomethyl)pyrrol-1-yl]butylamin als Öl.

$^1$H-NMR ($CD_3OD$): $\delta$ = 1,1-2,1 (m, 10H), 2,15-2,8 (m, 6H), 3,35 (s, 2H),

3,85 (t, 2H), 6,05 (m, 1H), 6,60 (m, 2H).

Analog Beispiel VI A wurden die Verbindungen der Beispiele VI B -VI V erhalten.

Verbindungen der Formel II, $R^3$=$R^4$=$R^5$=H

| Beispiel | $R^1$ | $R^2$ | -A- |
|---|---|---|---|
| VI B | $-(CH_2)_5-$ | | $-(CH_2)_2-$ |
| VI C | $-(CH_2)_5-$ | | $-(CH_2)_3-$ |
| VI D | $-(CH_2)_5-$ | | $-(CH_2)_5-$ |
| VI D | $-(CH_2)_5-$ | | $-(CH_2)_6-$ |
| VI F | $-(CH_2)_5-$ | | $-(CH_2)_8-$ |
| VI G | $-(CH_2)_5-$ | | $-(CH_2)_2-S-(CH_2)_2-$ |
| VI H | $-(CH_2)_5-$ | | $-(CH_2)_2-O-(CH_2)_2-$ |
| VI J | $C_6H_5CH_2-$ | $CH_3-$ | $-(CH_2)_5-$ |
| VI K | $n-C_4H_9-$ | $n-C_4H_9-$ | $-(CH_2)_4-$ |
| VI L | $C_6H_5CH_2-$ | $CH_3-$ | $-(CH_2)_4-$ |
| VI M | $-(CH_2)_4-$ | | $-(CH_2)_4-$ |
| VI N | $-(CH_2)_6-$ | | $-(CH_2)_4-$ |
| VI O | $-(CH_2)_2-CH(C_6H_5)-(CH_2)_2-$ | | $-(CH_2)_4-$ |
| VI P | $-CH_2-(o-C_6H_4)-CH_2-CH_2-$ | | $-(CH_2)_4-$ |
| VI Q | $CH_3-$ | $CH_3-$ | $-(CH_2)_5-$ |
| VI R | $-(CH_2)_2-CH(C_6H_5)-(CH_2)_2-$ | | $-(CH_2)_5-$ |
| VI S | $-CH_2-CH(C_6H_5)-(CH_2)_2-$ | | $-(CH_2)_5-$ |
| VI T | $-CH_2-(o-C_6H_4)-CH_2-CH_2-$ | | $-(CH_2)_5-$ |
| VI U | $C_6H_5CH_2-$ | $CH_3-$ | m-Xylylen |
| VI V | $-(CH_2)_5-$ | | m-Xylylen |

Beispiel VI AA

Eine Lösung von 3,6 g (14,3 mMol) der Verbindung des Beispiels II AA und 14,3 ml 1 N NaOH in 30 ml Methanol wurden 1 Tag bei Raumtemperatur gerührt. Anschließend wurde nach Zusatz von weiteren 14,3 ml 1 N NaOH 2 Tage bei 40 °C gerührt. Nach Abziehen des Methanols wurde mit Methylenchlorid extrahiert, der organische Extrakt über $Na_2SO_4$ getrocknet und am Rotationsverdampfer eingeengt. Man erhielt 2,4 g 4-[3-(Isobutylaminomethyl)pyrrol-1-yl]-butylamin als Öl.

$^1H$-NMR ($d_6$-DMSO): $\delta$ = 0,85 (d, 6H), 1,1-2,0 - (m, 7H), 2,36 (d, 2H),

2,57 (t, 2H), 3,50 (s, 2H), 3,81 (t, 2H),

5,95 (m, 1H), 6,63 (m, 2H).

Beispiele VI AB -VI AD

Analog Beispiel VI AA wurden die Verbindungen der Beispiele VI AB -VI AD hergestellt.

Verbindung der Formel II, $R^3=R^4=R^5=H$

| Beispiel | $R^1$ | $R^2$ | -A- |
|---|---|---|---|
| VI AB | $c-C_6H_{11}-$ | H | $-(CH_2)_4-$ |
| VI AC | $C_6H_5-CH_2-$ | H | $-(CH_2)_4-$ |
| VI AD | $n-C_{12}H_{25}-$ | H | $-(CH_2)_5-$ |

Beispiele VI BA -VI BC

Analog Beispiel VI A wurden die Verbindungen der Beispiele VI BA -VI BC erhalten.

Verbindungen der Formel II, $R^3=R^5=H$, $R^4=CH_3$

<table>
<tr><th>Beispiel</th><th>$R^1$</th><th>$R^2$</th><th>-A-</th></tr>
<tr><td>VI BA</td><td colspan="2">$-(CH_2)_5-$</td><td>$-(CH_2)_4-$</td></tr>
<tr><td>VI BB</td><td>$C_6H_5CH_2-$</td><td>$CH_3-$</td><td>$-(CH_2)_5-$</td></tr>
<tr><td>VI BC</td><td colspan="2">$-(CH_2)_5-$</td><td>$-(CH_2)_5-$</td></tr>
</table>

Beispiele VII A

Eine Lösung von 2 g (4,9 mMol) der Verbindung des Beispiels III B in 50 ml Essigsäure wurde in Gegenwart von 0,3 g Pd/Kohle (10 r) drucklos bei Raumtemperatur hydriert. Nach Abfiltrieren des Katalysators wurde die Lösung am Rotationsverdampfer eingeengt, mit NaOH alkalisch gestellt und mit $CH_2Cl_2$ extrahiert. Man erhielt 1,0 g 4-[3-(N-Benzyl-N-methylamino)-pyrrol-1-yl]butylamin als Öl.

$^1H$-NMR ($CDCl_3$): $\delta$ = 1,1 (br.s, 2H), 1,40 (m, 2H), 1,75 (m, 2H),

2,20 (s, 3H), 2,68 (t, 2H), 3,44 (s, 2H),

3,48 (s, 2H), 3,85 (t, 2H), 6,08 (m, 1H),

6,55 (m, 2H), 7,3 (m, 5H).

Beispiele VIII A -VIII C

Analog Beispiel VI a wurden die Verbindungen der Beispiele VIII A bis VIII C erhalten.

Verbindungen der Formel II, $R^3=R^4=CH_3$, $R^5=H$

| Beispiel | $R^1$ | $R^2$ | -A- |
|---|---|---|---|
| VIII A | $-(CH_2)_5-$ | | $-(CH_2)_5-$ |
| VIII B | $CH_3-$ | $CH_3-$ | $-(CH_2)_5-$ |
| VIII C | $C_6H_5CH_2-$ | $CH_3-$ | $-(CH_2)_5-$ |

Beispiele IX A -IX E

Verbindungen der Formel II,

$R^3=H$, $R^4+R^5$ = 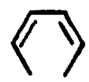

Beispiel IX A

Eine Mischung aus 38,3 g (86,5 mMol) der Verbindung aus Beispiel V A und 8,7 g (173 mMol) Hydrazinhydrat wurde 6 Stunden am Rückfluß gekocht. Nach Abkühlen auf Raumtemperatur wurde der Niederschlag abfiltriert, mit Ethanol gewaschen und die vereinigten Filtrate am Rotationsverdampfer eingeengt. Nach Zugabe von Wasser wurde mit 2 N HCl auf pH 2 eingestellt, die ausgefallenen Kristalle abgesaugt und das Filtrat mit Essigester mehrmals extrahiert. Anschließend wurde die wäßrige Phase mit 2 N NaOH alkalisch gestellt und mit Methylenchlorid extrahiert. Nach Trocknen über $Na_2SO_4$ und Abziehen des Lösungsmittels wurden 20,8 g 6-[3-(Piperidinomethyl)indol-1-yl]-hexylamin als Öl erhalten.

$^1$H-NMR ($d_6$-DMSO): $\delta$ = 1,0-1,9 (m, 14H), 2,0 - (br.s, 2H), 2,1-2,7 (m, 8H),

3,6 (br.s, 2H), 4,1 (t, 2H), 6,9-7,8 (m, 5H).

Beispiele IX B -IX E

Analog Beispiel IX A wurden die Verbindungen der Beispiele IX B -IX E erhalten.

Verbindungen der Formel II, $R^3=H$, $R^4+R^5$ = 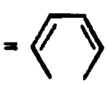

| Beispiel | $R^1$ | $R^2$ | A |
|---|---|---|---|
| IX B | $-(CH_2)_5-$ | | $-(CH_2)_4-$ |
| IX C | $-(CH_2)_5-$ | | $-(CH_2)_5-$ |
| IX D | $CH_3-$ | $CH_3-$ | $-(CH_2)_5-$ |
| IX E | $C_6H_5CH_2-$ | $CH_3-$ | $-(CH_2)_5-$ |

Beispiele XA -XD

Verbindungen der Formel XIV

Analog Beispiel IM können durch Umsetzung der entsprechenden Mono(pyridin-3-yl-methyl)-oxycarbonyldiaminoalkane mit 2.5-Dimethoxytetrahydrofuran-3-carbaldehyd die Verbindungen der Beispiele XA -XD erhalten werden.

Verbindungen der Formel XIV, $R^3=R^4=R^5=H$

| Beispiel | -A- | -NQ |
|---|---|---|
| XA | $-(CH_2)_4-$ | $-NHCOOCH_2-$(pyridin-3-yl) |
| XB | $-(CH_2)_5-$ | $-NHCOOCH_2-$(pyridin-3-yl) |
| XC | $-(CH_2)_4-$ | $-NHCOOCH_2-$(6-methylpyridin-3-yl) |
| XD | $-(CH_2)_5-$ | $-NHCOOCH_2-$(6-methylpyridin-3-yl) |

Beispiele XI A -XI H

Verbindungen der Formel XV

Durch Umsetzung der Verbindungen der Beispiele XA -XD mit Aminen analog Beispiel III A können die Verbindungen der Beispiele XI A -XI H erhalten werden.

Verbindungen der Formel XV, $R^3=R^4=R^5=H$

| Beispiel | $R^1$ | $R^2$ | -A- | NQ |
|---|---|---|---|---|
| XI A | $C_6H_5CH_2$ | $CH_3$ | $-(CH_2)_4-$ | $NHCOOCH_2-$(pyridin-3-yl) |
| XI B | $C_6H_5CH_2$ | $CH_3$ | $-(CH_2)_5-$ | $NHCOOCH_2-$(pyridin-3-yl) |
| XI C | $-(CH_2)_5-$ | | $-(CH_2)_4-$ | $NHCOOCH_2-$(pyridin-3-yl) |
| XI D | $-(CH_2)_5-$ | | $-(CH_2)_5-$ | $NHCOOCH_2-$(pyridin-3-yl) |
| XI E | $C_6H_5CH_2$ | $CH_3$ | $-(CH_2)_4-$ | $NHCOOCH_2-$(6-methylpyridin -3-yl |
| XI F | $C_6H_5CH_2$ | $CH_3$ | $-(CH_2)_5-$ | $NHCOOCH_2-$(6-methylpyridin -3-yl) |
| XI G | $-(CH_2)_5-$ | | $-(CH_2)_4-$ | $NHCOOCH_2-$(6-methylpyridin -3-yl) |
| XI H | $-(CH_2)_5-$ | | $-(CH_2)_5-$ | $NHCOOCH_2-$(6-methylpyridin -3-yl) |

Durch katalytische Hydrierung der Verbindungen der Beispiele XI A -XI H analog Beispiel VII A können die Verbindungen der Formel II der Beispiele VI A, VI D, VI J und VI L erhalten werden.

Herstellungsbeispiele für erfindungsgemäße Verbindungen

Beispiele 1 -4

Verbindungen der Formel I, $R^3=R^4=R^5=H$, $B=B'$, $E=NCN$

Beispiel 1

Eine Lösung von 2,85 g (10 mMol) der Verbindung des Beispiels VI J in 50 ml Methanol wurde mit 1,5 g (10 mMol) Cyaniminodimethyldithiocarbonat versetzt und zwei Stunden bei Raumtemperatur und vier Stunden bei 50 °C gerührt. Anschließend wurde bei Raumtemperatur die Reaktionslösung, die das Zwischenprodukt der Formel IV, R' = $C_6H_5CH_2$, $R^2 = CH_3$, $R^3 = R^4 = R^5 = H$, -A- = $-(CH_2)_5-$, E = NCN, Y' = $SCH_3$ enthält, mit 4 g (50 mMol) 40prozentiger Methylaminlösung versetzt und 3 Tage bei 50 °C nachgerührt. Nach Abziehen des Lösungsmittels wurde der Rückstand über Kieselgel säulenchromatographiert ($CH_2Cl_2/CH_3OH$ 4 : 1). Die produkthaltigen Fraktionen wurden abrotiert und das Produkt durch Umsetzung mit 1 Äquivalent Weinsäure in 30 ml Methanol und Abziehen des Lösungsmittels in das Tartrat übergeführt. Man erhielt 2,5 g N-{5-[3(-N-Benzyl-N-methylaminomethyl)pyrrol-1-yl]pentyl }-N'-cyano-N"-methylguanidin• 1 Weinsäure als schaumartiges Festprodukt.

$^1$H-NMR ($d_6$-DMSO): $\delta$ = 1,25 (m, 2H), 1,50 (m, 2H), 1,75 (m, 2H),

2,40 (s, 3H), 2,68 (d, 3H), 3,1 (m,2H),

3,90 (m, 2H), 3,90 (s, 2H), 4,00 (2, 2H),

4,2 (s, 2H), 6,15 (m, 1H), 6,8 (m, 1H),

6,95 (m, 3H), 7,45 (m, 3H).

Beispiele 2 -3

Analog Beispiel 1 wurden die Verbindungen der Beispiele 2 und 3 hergestellt.

Verbindungen der Formel I, $R^3 = R^4 = R^5 = H$, B= $-\overset{NCN}{\overset{\|}{C}}-NHCH_3$

| Beispiel | $R^1$ | $R^2$ | -A- |
|---|---|---|---|
| 2 | $-(CH_2)_5-$ | | $-(CH_2)_5-$ |
| 3 | $CH_3-$ | $CH_3-$ | $-(CH_2)_5-$ |

Beispiel 4

Durch Umsetzung von 2,1 g (10 mMol) der Verbindung des Beispiels VI Q mit 2,1 g (10 mMol) S-Methyl-N-(3-Pyridylmethyl)-cyaniminothiocarbaminsäureester bei 80 °C im Vakuum (3 Stunden) wurde nach Chromatographie über Kieselgel und Umsetzung mit 1 Äquivalent Weinsäure in Methanol 2,5 g N-[5-(3-Dimethylaminomethylpyrrol-1-yl-)pentyl]-N'-cyano-N"-(3-pyridylmethyl)-guanidin• 1 Weinsäure als schaumartiges Festprodukt erhalten.

$^1$H-NMR ($d_6$-DMSO): $\delta$ = 1,15 (m, 2H), 1,45 (m, 2H), 1,65 (m, 2H),

2,17 (s, 6H), 3,10 (m, 2H), 3,85 (t, 2H),

3,95 (s, 2H), 4,05 (s, 2H), 4,35 (d, 2H),

6,10 (m, 1H), 6,78 (m, 1H), 6,90 (m, 1H),

7,18 (m, 1H), 7,4 (m, 1H), 7,67 (m, 2H),

8,50 (m, 2H).

Beispiele 5 -19

Verbindungen der Formel I, B = B', E = $-CH-NO_2$

Beispiel 5

Eine Mischung von 2,0 g (7,5 mMol) der Verbindung aus Beispiel VI L und 1,1 g (7,5 mMol) 2-Methylamino-2-methylthio-nitroethylen wurde im Vakuum 2 Stunden bei 80 °C gerührt. Nach Chromatographie über Kieselgel ($CH_2Cl_2/CH_3OH$ 4 : 1) wurde das erhaltene Öl mit 1 Äquivalent Weinsäure in 30 ml Ethanol umgesetzt. Nach Abziehen des

Lösungsmittels wurden 2,0 g 2-{4-[3-(N-Benzyl-N-methylaminomethyl)pyrrol-1-yl-]butylamino}-2-methylamino-1-nitroethylen • 1 Weinsäure als - schaumartige Festsubstanz isoliert.

$^1$H-NMR ($d_6$-DMSO): $\delta$ = 1,46 (2H), 1,75 (m, 2H), 2,45 (s, 3H),

2,8 (m, 3H), 3,2 (m, 2H), 3,93 (m, 2H),

3,98 (s, 2H), 4,07 (s, 2H), 4,23 (s, 2H),

6,17 (m, 1H), 6,45 (m, 1H), 6,80 (m, 1H),

6,95 (m, 1H), 7,35-7,60 (m, 5H).

Beispiel 6 - 18

Analog Beispiel 5 wurden die Verbindungen der Beispiele 6 - 18 erhalten.

Verbindungen der Formel I, B=$B^1$, E=$CH-NO_2$

| Beispiele | $R^1$ | $R^2$ | $R^3$ | $R^4$ | $R^5$ | -A- | $R^6$ |
|---|---|---|---|---|---|---|---|
| 6 | $-(CH_2)_6-$ | | H | H | H | $-(CH_2)_4-$ | $CH_3-$ |
| 7 | $-CH_2-(o-C_6H_4)-CH_2-CH_2-$ | | H | H | H | $-(CH_2)_4-$ | $CH_3-$ |
| 8 | $-(CH_2)_2-CH(C_6H_5)-(CH_2)_2-$ | | H | H | H | $-(CH_2)_4-$ | $CH_3-$ |
| 9 | $-(CH_2)_5-$ | | H | H | H | $-(CH_2)_5-$ | $CH_3-$ |
| 10 | $C_6H_5CH_2-$ | $CH_3-$ | H | H | H | $-(CH_2)_5-$ | $CH_3-$ |
| 11 | $C_6H_5CH_2-$ | $CH_3-$ | H | H | H | m-Xylylen | $CH_3-$ |
| 12 | $-(CH_2)_5-$ | | H | $CH_3-$ | H | $-(CH_2)_5-$ | $CH_3-$ |
| 13 | $C_6H_5CH_2-$ | $CH_3-$ | H | $CH_3-$ | H | $-(CH_2)_5-$ | $CH_3-$ |
| 14 | $-(CH_2)_5-$ | | $CH_3-$ | $CH_3-$ | H | $-(CH_2)_5-$ | $CH_3-$ |
| 15 | $C_6H_5CH_2-$ | $CH_3-$ | $CH_3-$ | $CH_3-$ | H | $-(CH_2)_5-$ | $CH_3-$ |
| 16 | $-(CH_2)_5-$ | | H | 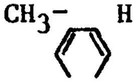 | | $-(CH_2)_5-$ | $CH_3-$ |
| 17 | $-(CH_2)_5-$ | | H | 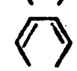 | | $-(CH_2)_4-$ | $CH_3-$ |
| 18 | $CH_3-$ | $CH_3-$ | H | H | H | $-(CH_2)_5-$ | (Pyridin-3-yl)-$CH_2-$ |

Beispiel 19

a) Durch Reaktion von 2,97 g (10 mMol) der Verbindung aus Beispiel VI T mit 6,6 g (40 mMol) 2,2-Bismethylthionitroethylen in 50 ml Methanol bei 50 °C (6 Stunden) wurde eine Lösung erhalten, die 2{5-[3-Tetrahydroisochinolin-2-ylmethyl)pyrrol-1-yl]pentylamino}2-methylthio-1-nitroethylen enthielt.

b) Diese Lösung wurde mit 15,5 g (200 mMol) 40prozentiger Methylaminlösung versetzt und 10 Stunden bei 50 °C gerührt. Nach Abziehen des Lösungsmittels und Chromatographie über Kieselgel ($CH_2Cl_2/CH_3OH$ 4 : 1) wurde das Produkt durch Zugabe von 1 Äquivalent Weinsäure in Methanol und Abziehen des Lösungsmittels in das Tartrat übergeführt. Man erhielt 2-{5-[3-(Tetrahydroisochinolin-2-ylmethyl)pyrrol-1-yl]-pentylamin}-2-methylamino-1-nitroethylen • 1 Weinsäure.

$^1$H-NMR ($d_6$-DMSO): δ = 1,30 (m, 2H), 1,58 (m, 2H), 1,75 (m, 2H),

2,8 (m, 3H), 3,0 (m, 2H), 3,2 (m, 4H),

3,90 (t, 2H), 4,00 (s, 2H), 4,08 (s, 2H),

4,20 (s, 2H), 6,15 (m, 1H), 6,50 (m, 1H),

6,85 (m, 1H), 6,95 (m, 1H), 7,25 (m, 4H).

Beispiel 20 -26

Verbindungen der Formel I, B = $B^2$

Beispiel 20

a) Eine Mischung von 8,0 g (30 mMol) der Verbindung des Beispiels VI L und 7,0 g (30 mMol) der Verbindung der Formel VI, $Y^3 = CH_3S$, $Z^2 = CN$, $R^7 = CH_3$ wurde im Vakuum 3 Stunden bei 100 °C umgesetzt. Nach Chromatographie über Kieselgel wurden 12,7 g des Zwischenprodukts der Formel VII, $R^1 = C_6H_5\text{-}CH_2$, $R^2 = CH_3$, $R^3 = R^4 = R^5 = H$, A = $-(CH_2)_4-$, $Z^2 = CN$, $R^7 = CH_3$ als Öl erhalten.

b) 12,7 g des in Beispiel 20 a) erhaltenen Produkts wurden mit 10,5 g Weinsäure in 150 ml Aceton und 150 ml Wasser über Nacht bei Raumtemperatur gerührt. Anschließend wurde mit Essigester extrahiert, die organische Phase verworfen und die Wasserphase mit 2 N NaOH alkalisiert und mit $CH_2Cl_2$ extrahiert. Nach Trocknen über $Na_2SO_4$ und Abziehen des Lösungsmittels wurde das Rohprodukt über Kieselgel chromatographiert ($CH_2Cl_2/CH_3OH$ 4 : 1). Man erhielt 4,7 g 5-{4-[3-(N-Benzyl-N-methylaminomethyl)pyrrol-1-yl]-butylamino}-3-amino-1-methyltri azol als Öl, das mit 1 Äquivalent Weinsäure in Methanol und Abziehen des Lösungsmittels in das Tartrat übergeführt wurde.

$^1$H-NMR ($d_6$-DMSO): δ = 1,45 (m, 2H), 1,7 (m, 2H), 2,23 (s, 3H),

3,1 (m, 2H), 3,24 (s, 3H), 3,64 (s, 2H),

3,75 (s, 2H), 3,88 (t, 2H), 4,10 (s, 2H), 6,03 (m, 1H), 6,10 (m, 1H), 6,75 (m, 1H),

6,80 (m, 1H), 7,35 (m, 5H).

Analog Beispiel 20 wurden die Verbindungen der Beispiele 21 -26 erhalten.

Verbindungen der Formel I, B=$B^2$, $R^7$=$CH_3$, $R^8$=$NH_2$

| Beispiel | $R^1$ | $R^2$ | $R^3$ | $R^4$ | $R^5$ | -A- |
|---|---|---|---|---|---|---|
| 21 | $-(CH_2)_2-CH(C_6H_5)-(CH_2)_2-$ | | H | H | H | $-(CH_2)_4-$ |
| 22 | $-CH_2(o-C_6H_4)-CH_2-CH_2-$ | | H | H | H | $-(CH_2)_4-$ |
| 23 | $-(CH_2)_5-$ | | H | H | H | $-(CH_2)_5-$ |
| 24 | $C_6H_5CH_2-$ | $CH_3-$ | H | H | H | $-(CH_2)_5-$ |
| 25 | $C_6H_5CH_2-$ | $CH_3-$ | H | $CH_3$ | H | $-(CH_2)_5-$ |
| 26 | $-(CH_2)_5-$ | | H | H | H | m-Xylylen |

Beispiele 27 -85

Verbindungen der Formel I, B = $B^5$

Beispiel 27

Eine Mischung aus 1,6 g (6,8 mMol) 4-[3-(Piperidin-1-yl-methyl)pyrrol-1-yl]butylamin (Beispiel VI A) und 1,0 g (6,8 mMol) 4-Methoxy-3-amino-1.2.5-thiadiazol-S-oxid in 15 ml Methanol wurde über Nacht bei Raumtemperatur gerührt. Nach Abziehen des Lösungsmittels wurde das Rohprodukt durch eine Säulenchromatographie über Kieselgel ($CH_2Cl_2/CH_3OH$ 4 : 1) gereinigt. Nach Umsetzung mit 1 Äquivalent Weinsäure in Ethanol wurde nach Abziehen des Lösungsmittels 2,0 g 3-{4-[3-(Piperidin-1-yl-methyl)pyrrol-1-yl]-butylamino}-4-amino-1.2.5-thiadiazol-S-oxid • 1 Weinsäure erhalten.

$^1$H-NMR ($d_6$-DMSO): $\delta$ = 1,55 + 1,75 (m, 10H), 3,0 (m, 4H), 3,32 (m, 2H),

3,95 (t, 2H), 4,0 (s, 2H), 4,18 (s, 2H),

6,13 (m, 1H), 6,85 (m, 1H), 6,97 (m, 1H).

Analog Beispiel 27 wurden die Verbindungen der Beispiele 28 - 85 erhalten.

Beispiele 28 -69

Verbindungen der Formel I, $R^3=R^4=R^5=H$, $B=B^5$, $m=1$;

| Beispiel | $R^1$ | $R^2$ | -A- | $R^{11}$ |
|---|---|---|---|---|
| 28 | $-(CH_2)_5-$ | | $-(CH_2)_2-$ | H |
| 29 | $-(CH_2)_5-$ | | $-(CH_2)_2-$ | $CH_3$ |
| 30 | $-(CH_2)_5-$ | | $-(CH_2)_3-$ | H |
| 31 | $-(CH_2)_5-$ | | $-(CH_2)_3-$ | $CH_3$ |
| 32 | $-(CH_2)_5-$ | | $-(CH_2)_4-$ | $CH_3$ |
| 33 | $-(CH_2)_5-$ | | $-(CH_2)_5-$ | H |
| 34 | $-(CH_2)_5-$ | | $-(CH_2)_5-$ | $CH_3$ |
| 35 | $-(CH_2)_5-$ | | $-(CH_2)_6-$ | H |
| 36 | $-(CH_2)_5-$ | | $-(CH_2)_6-$ | $CH_3$ |
| 37 | $-(CH_2)_5-$ | | $-(CH_2)_8-$ | H |
| 38 | $-(CH_2)_5-$ | | $-(CH_2)_8-$ | $CH_3$ |
| 39 | $-(CH_2)_5-$ | | $-(CH_2)_2-S-(CH_2)_2-$ | H |
| 40 | $-(CH_2)_5-$ | | $-(CH_2)_2-S-(CH_2)_2-$ | $CH_3$ |
| 41 | $-(CH_2)_5-$ | | $-(CH_2)_2-O-(CH_2)_2-$ | H |
| 42 | $C_6H_5CH_2$ | $CH_3$ | $-(CH_2)_4-$ | H |
| 43 | $C_6H_5CH_2$ | $CH_3$ | $-(CH_2)_4-$ | $CH_3$ |
| 44 | $-(CH_2)_2-CH(C_6H_5)-(CH_2)_2-$ | | $-(CH_2)_4-$ | H |
| 45 | $-CH_2-(o-C_6H_4)-CH_2-CH_2-$ | | $-(CH_2)_4-$ | H |
| 46 | $(CH_3)_2CH-CH_2-$ | H | $-(CH_2)_4-$ | $CH_3$ |
| 47 | Cyclohexyl | H | $-(CH_2)_4-$ | H |
| 48 | $-(CH_2)_4-$ | | $-(CH_2)_4-$ | H |
| 49 | $-(CH_2)_4-$ | | $-(CH_2)_4-$ | $CH_3$ |
| 50 | $-(CH_2)_6-$ | | $-(CH_2)_4-$ | H |
| 51 | $-(CH_2)_6-$ | | $-(CH_2)_4-$ | $CH_3$ |
| 52 | $C_6H_5CH_2$ | H | $-(CH_2)_4-$ | H |
| 53 | $C_6H_5CH_2$ | H | $-(CH_2)_4-$ | $CH_3$ |
| 54 | $n-C_4H_9$ | $n-C_4H_9$ | $-(CH_2)_4-$ | H |
| 55 | $CH_3$ | $CH_3$ | $-(CH_2)_5-$ | H |
| 56 | $CH_3$ | $CH_3$ | $-(CH_2)_5-$ | $CH_3$ |
| 57 | $CH_3$ | $CH_3$ | $-(CH_2)_5-$ | (3-Pyridyl)-$CH_2-$ |
| 58 | $-(CH_2)_9-$ | | $-(CH_2)_5-$ | H |
| 59 | $-(CH_2)_9-$ | | $-(CH_2)_5-$ | $CH_3$ |
| 60 | $-(CH_2)_5-$ | | $-(CH_2)_5-$ | (3-Pyridyl)-$CH_2$ |
| 61 | $C_6H_5CH_2$ | $CH_3$ | $-(CH_2)_5-$ | H |

| Beispiel | $R^1$ | $R^2$ | -A- | $R^{11}$ |
|---|---|---|---|---|
| 62 | $C_6H_5CH_2$ | $CH_3$ | $-(CH_2)_5-$ | $CH_3$ |
| 63 | $C_6H_5CH_2$ | $CH_3$ | $-(CH_2)_5-$ | (3-Pyridyl)-$CH_2$- |
| 64 | $-(CH_2)_2-CH(C_6H_5)-(CH_2)_2-$ | | $-(CH_2)_5-$ | H |
| 65 | $-CH_2-(o-C_6H_4)-CH_2-CH_2-$ | | $-(CH_2)_5-$ | H |
| 66 | $CH_3$ | $CH_3$ | $-(CH_2)_5-$ | $-CH_2-CH_2-CH_2-$ 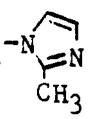 |
| 67 | $-CH_2-CH(C_6H_5)-(CH_2)_2-$ | | $-(CH_2)_5-$ | H |
| 68 | n-$C_{12}H_{25}$ | H | $-(CH_2)_5-$ | H |
| 69 | $-(CH_2)_5-$ | | m-Xylylen | H |

Beispiele 70 -85

Verbindungen der Formel I, $B=B^5$, $m = 1$

| Beispiel | $R^1$ | $R^2$ | $R^3$ | $R^4$ | $R^5$ | -A- | $R^{11}$ |
|---|---|---|---|---|---|---|---|
| 70 | $-(CH_2)_5-$ | | H | $CH_3$ | H | $-(CH_2)_4-$ | H |
| 71 | $-(CH_2)_5-$ | | H | $CH_3$ | H | $-(CH_2)_4-$ | $CH_3$ |
| 72 | $-(CH_2)_5-$ | | H | $CH_3$ | H | $-(CH_2)_5-$ | H |
| 73 | $C_6H_5CH_2$ | $CH_3$ | H | $CH_3$ | H | $-(CH_2)_5-$ | H |
| 74 | $C_6H_5CH_2$ | $CH_3$ | H | $CH_3$ | H | $-(CH_2)_5-$ | $CH_3$ |
| 75 | $CH_3$ | $CH_3$ | $CH_3$ | $CH_3$ | H | $-(CH_2)_5-$ | H |
| 76 | $CH_3$ | $CH_3$ | $CH_3$ | $CH_3$ | H | $-(CH_2)_5-$ | $CH_3$ |
| 77 | $-(CH_2)_5-$ | | $CH_3$ | $CH_3$ | H | $-(CH_2)_5-$ | H |
| 78 | $-(CH_2)_5-$ | | $CH_3$ | $CH_3$ | H | $-(CH_2)_5-$ | $CH_3$ |
| 79 | $C_6H_5CH_2$ | $CH_3$ | $CH_3$ | $CH_3$ | H | $-(CH_2)_5-$ | H |
| 80 | $C_6H_5CH_2$ | $CH_3$ | $CH_3$ | $CH_3$ | H | $-(CH_2)_5-$ | $CH_3$ |
| 81 | $CH_3$ | $CH_3$ | H | 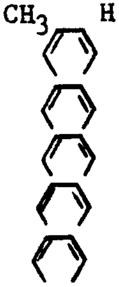 | | $-(CH_2)_5-$ | H |
| 82 | $-(CH_2)_5-$ | | H | | | $-(CH_2)_5-$ | H |
| 83 | $C_6H_5CH_2$ | $CH_3$ | H | | | $-(CH_2)_5-$ | H |
| 84 | $-(CH_2)_5-$ | | H | | | $-(CH_2)_4-$ | H |
| 85 | $-(CH_2)_5-$ | | H | | | $-(CH_2)_6-$ | H |

Beispiel 86

Zu einer Lösung von 4,02 g (15 mMol) der Verbindung aus Beispiel VII A in 100 ml absolutem Chloroform wurden bei Rückflußtemperatur eine Lösung von 3,02 g 3-Chlorbenzoisothiazol-S-S-dioxyd in 100 ml Chloroform zugetropft. Nach 30 Minuten Rückflußkochen wurde zur Trockne eingeengt und der Rückstand über Kieselgel chromatographiert ($CH_2Cl_2$/$CH_3OH$ 4 : 1).

Nach Umsetzen des reinen Produkts mit 1 Äquivalent Weinsäure wurden 2,0 g 3-{4-[3-(N-Benzyl-N-methyl-aminomethyl)-pyrrol-1-yl]-butylamino}benzisothiazol-S.S-dioxyd • 1 Weinsäure als schaumartige Festsubstanz erhalten.

$^1$H-NMR ($d_6$-DMSO): $\delta$ = 1,60 (m, 2H), 1,80 (m, 2H), 2,38 (s, 3H),

3,50 (m, 2H), 3,91 (s, 2H), 3,95 (m, 2H),

4,00 (s, 2H), 4,20 (s, 2H), 6,12 (m, 1H),

6,80 (m, 1H), 6,95 (m, 1H), 7,45 (m, 5H)

7,8 (m, 2H), 7,98 (m, 1H), 8,25 (m, 1H).

Beispiele 87 -90

Verbindungen der Formel I, B = B$^1$, E = S

Durch Umsetzung der Verbindungen der Formel II mit Methylisothiocyanat in THF können die Verbindungen der Beispiele 87 -90 erhalten werden.

Verbindungen der Formel I, $R^3$=$R^4$=$R^5$=H, B=$B^1$, E=S, $R^6$=$CH_3$

| Beispiel | $R^1$ | $R^2$ | -A- |
|---|---|---|---|
| 87 | $-(CH_2)_5-$ | | $-(CH_2)_4-$ |
| 88 | $-(CH_2)_5-$ | | $-(CH_2)_5-$ |
| 89 | $C_6H_5CH_2$ | $CH_3$ | $-(CH_2)_4-$ |
| 90 | $C_6H_5CH_2$ | $CH_3$ | $-(CH_2)_5-$ |

Beispiele 91 -95

Verbindungen der Formel I, B = B$^1$, E = NCN

Analog Beispiel 1 können die Verbindungen der Beispiele 91 -95 erhalten werden.

Verbindungen der Formel I, $R^3$=$R^4$=$R^5$=H, B=$B^1$, E=NCN

| Beispiel | $R^1$ | $R^2$ | -A- | $R^6$ |
|---|---|---|---|---|
| 91 | $-(CH_2)_5-$ | | $-(CH_2)_4-$ | $CH_3$ |
| 92 | $C_6H_5-CH_2$ | $CH_3$ | $-(CH_2)_4-$ | H |
| 93 | n-$C_4H_9$ | n-$C_4H_9$ | $-(CH_2)_4-$ | $CH_3$ |
| 94 | $C_6H_5CH_2$ | $CH_3$ | $-(CH_2)_5-$ | $CH_2CH_2OH$ |
| 95 | $-(CH_2)_5-$ | | $-(CH_2)_4-$ | $CH_2CH_2OCH_3$ |

Beispiele 96 -123

Verbindungen der Formel I, $R^3$ = $R^5$ = H, B = B$^1$, E = $CHNO_2$

Analog Beispiel 5 können die Verbindungen der Beispiele 96 -123 erhalten werden.

| Beispiel | $R^1$ | $R^2$ | $R^4$ | -A- | $R^6$ |
|---|---|---|---|---|---|
| 96 | (2-Naphthyl-)$CH_2$- | $CH_3$ | H | $-(CH_2)_4-$ | $CH_3$ |
| 97 | (3.4-Dimethoxyphenyl)-$CH_2$-$CH_2$- | $CH_3$ | H | $-(CH_2)_4-$ | $CH_3$ |
| 98 | $C_6H_5-CH_2$ | $CH_3$ | H | $-(CH_2)_4-$ | $-CH_2-CH_2-OH$ |
| 99 | $C_6H_5-CH_2$ | $CH_3$ | H | $-(CH_2)_5-$ | $-CH_2-CH_2-OH$ |
| 100 | 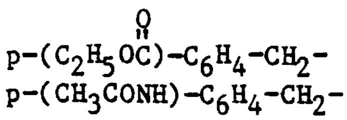 | $CH_3$ | H | $-(CH_2)_5-$ | $CH_3$ |
| 101 | p-($CH_3CONH$)-$C_6H_4$-$CH_2$- | $CH_3$ | H | $-(CH_2)_4-$ | $CH_3$ |
| 102 | (Pyridin-3-yl)$CH_2$- | H | H | $-(CH_2)_4-$ | $CH_3$ |
| 103 | (2-Furyl)-$CH_2$- | $CH_3$ | H | $-(CH_2)_4-$ | $CH_3$ |
| 104 | (2-Thienyl)-$CH_2$- | $CH_3$ | H | $-(CH_2)_4-$ | $CH_3$ |
| 105 | (3-Thienyl)-$CH_2$- | $CH_3$ | H | $-(CH_2)_4-$ | $CH_3$ |
| 106 | (1-Methylpyrrol-2-yl)-$CH_2$- | $CH_3$ | H | $-(CH_2)_4-$ | $CH_3$ |
| 107 | (1-Methylpyrrol-3-yl)-$CH_2$ | $CH_3$ | H | $-(CH_2)_4-$ | $CH_3$ |
| 108 | (1-Methylpyrazol-(4-yl)-$CH_2$ | $CH_3$ | H | $-(CH_2)_4-$ | $CH_3$ |
| 109 | $-(CH_2)_{11}-$ | | H | $-(CH_2)_4-$ | $CH_3$ |
| 110 | 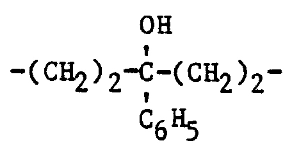 | | H | $-(CH_2)_5-$ | $CH_3$ |
| 111 | 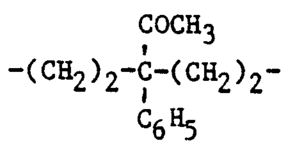 | | H | $-(CH_2)_4-$ | $CH_3$ |
| 112 | 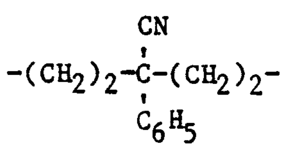 | | H | $-(CH_2)_4-$ | $CH_3$ |
| 113 | 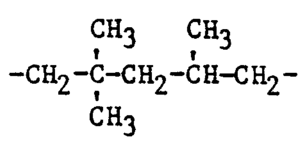 | | H | $-(CH_2)_4-$ | H |

| Beispiel | $R^1$ | $R^2$ | $R^4$ | -A- | $R^6$ |
|---|---|---|---|---|---|
| 114 | 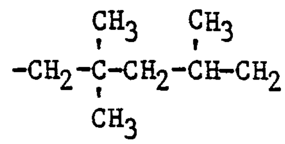 | | H | $-(CH_2)_4-$ | $CH_3$ |
| 115 | 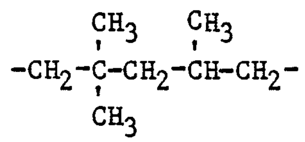 | | H | $-(CH_2)_4-$ | $-CH_2-CH_2-OH$ |
| 116 | $-(CH_2)_5-$ | | $CH_3$ | $-(CH_2)_4-$ | $CH_3$ |
| 117 | $C_6H_5CH_2$ | $CH_3$ | $CH_3$ | $-(CH_2)_4-$ | $CH_3$ |
| 118 | $n-C_4H_9$ | $n-C_4H_9$ | H | $-(CH_2)_4-$ | $CH_3$ |
| 119 | $n-C_4H_9$ | $n-C_4H_9$ | H | $-(CH_2)_5-$ | $CH_3$ |
| 120 | $n-C_{12}H_{25}$ | H | H | $-(CH_2)_4-$ | $CH_3$ |
| 121 | $n-C_{12}H_{25}$ | H | H | $-(CH_2)_5-$ | $CH_3$ |
| 122 | $n-C_{12}H_{25}$ | $CH_3$ | H | $-(CH_2)_4-$ | $CH_3$ |
| 123 | $n-C_{12}H_{25}$ | $CH_3$ | H | $-(CH_2)_5-$ | $CH_3$ |

Beispiele 124 -152

Verbindungen der Formel I, $B = B^2$

Analog Beispiel 20 können die Verbindungen der Beispiele 124 -133 erhalten werden.

Verbindungen der Formel I, $R^5$=H, B=$B^2$, $R^8$=$NH_2$

| Beispiel | $R^1$ | $R^2$ | $R^3$ | $R^4$ | A | $R^7$ |
|---|---|---|---|---|---|---|
| 124 | $-(CH_2)_5-$ | | H | H | $-(CH_2)_4-$ | $CH_3$ |
| 125 | $-(CH_2)_5-$ | | H | $CH_3$ | $-(CH_2)_4-$ | $CH_3$ |
| 126 | $-(CH_2)_5-$ | | H | $CH_3$ | $-(CH_2)_5-$ | $CH_3$ |
| 127 | $-(CH_2)_5-$ | | $CH_3$ | $CH_3$ | $-(CH_2)_5-$ | $CH_3$ |
| 128 | $n-C_4H_9$ | $n-C_4H_9$ | H | H | $-(CH_2)_4-$ | $CH_3$ |
| 129 | $n-C_4H_9$ | $n-C_4H_9$ | H | H | $-(CH_2)_5-$ | $CH_3$ |
| 130 | $n-C_{12}H_{25}$ | H | H | H | $-(CH_2)_4-$ | $CH_3$ |
| 131 | $n-C_{12}H_{25}$ | $CH_3$ | H | H | $-(CH_2)_4-$ | $CH_3$ |
| 132 | $C_6H_5CH_2$ | $CH_3$ | H | $CH_3$ | $-(CH_2)_4-$ | $CH_3$ |
| 133 | $C_6H_5CH_2$ | $CH_3$ | H | H | $-(CH_2)_4-$ | $CH_2CH_2OH$ |

Beispiele 134 -137

Durch Umsetzung der Zwischenprodukte der Formel IV, $E = NCN$, $Y' = SCH_3$ mit Hydrazin in Ethanol können die Verbindungen der Beispiele 134 -137 erhalten werden.

Verbindungen der Formel I, $R^3=R^4=R^5=H$, $B=B^2$, $R^7=H$, $R^8=NH_2$

| Beispiel | $R^1$ | $R^2$ | -A- |
|---|---|---|---|
| 134 | $-(CH_2)_5-$ | | $-(CH_2)_4-$ |
| 135 | $-(CH_2)_5-$ | | $-(CH_2)_5-$ |
| 136 | $C_6H_5CH_2$ | $CH_3$ | $-(CH_2)_4-$ |
| 137 | $n-C_4H_9$ | $n-C_4H_9$ | $-(CH_2)_4-$ |

Beispiele 138 -146

Durch Umsetzung der Zwischenprodukte der Formel VI, $Y^3 = CH_3S$, $R^7 = CH_3$, $Z^2 = \overset{O}{\overset{\|}{C}}\ CH_2OR^{12'}$ mit Aminen der Formel II und anschließende Cyclisierung mit Weinsäure analog Beispiel 20 können die Verbindungen der Beispiele 138 -146 erhalten werden.

Verbindungen der Formel I, $R^3=R^4=R^5=H$, $B=B^2$, $R^7=CH_3$, $R^8=CH_2-O-R^{12'}$

| Beispiel | $R^1$ | $R^2$ | -A- | $R^{12}$ |
|---|---|---|---|---|
| 138 | $C_6H_5CH_2$ | $CH_3$ | $-(CH_2)_4-$ | $-COCH_3$ |
| 139 | $C_6H_5CH_2$ | $CH_3$ | $-(CH_2)_4-$ | $-CO-C_6H_5$ |
| 140 | $C_6H_5CH_2$ | $CH_3$ | $-(CH_2)_4-$ | Tetrahydropyran-2-yl |
| 141 | $C_6H_5CH_2$ | $CH_3$ | $-(CH_2)_4-$ | Tetrahydrofuran-2-yl |
| 142 | $C_6H_5CH_2$ | $CH_3$ | $-(CH_2)_5-$ | $-COCH_3$ |
| 143 | $-(CH_2)_5-$ | | $-(CH_2)_4-$ | $-COCH_3$ |
| 144 | $-(CH_2)_5-$ | | $-(CH_2)_5-$ | $-COCH_3$ |
| 145 | $n-C_4H_9$ | $n-C_4H_9$ | $-(CH_2)_4-$ | $-COCH_3$ |
| 146 | $n-C_4H_9$ | $n-C_4H_9$ | $-(CH_2)_5-$ | $-COCH_3$ |

Beispiel 147 -152

Durch alkalische Hydrolyse der Verbindungen der Beispiele 138, 142 -146 können die Verbindungen der Beispiele 147 -152 erhalten werden.

Verbindungen der Formel I, $R^3$=$R^4$=$R^5$=H, B=$B^2$, $R^7$=$CH_3$, $R^8$=$CH_2OH$

| Beispiel | $R^1$ | $R^2$ | -A- |
|---|---|---|---|
| 147 | $C_6H_5CH_2$ | $CH_3$ | $-(CH_2)_4-$ |
| 148 | $C_6H_5CH_2$ | $CH_3$ | $-(CH_2)_5-$ |
| 149 | $-(CH_2)_5-$ | | $-(CH_2)_4-$ |
| 150 | $-(CH_2)_5-$ | | $-(CH_2)_5-$ |
| 151 | $n-C_4H_9$ | $n-C_4H_9$ | $-(CH_2)_4-$ |
| 152 | $n-C_4H_9$ | $n-C_4H_9$ | $-(CH_2)_5-$ |

Beispiele 153 -160

Verbindungen der Formel I, B = $B^3$

Durch Umsetzung der Amine der Formel II mit Verbindungen der Formel VIII, $Y^2$ = Cl, $Z^3$ = $OCH_2C_6H_5$ und anschließende katalytische Abspaltung der Benzylgruppe analog den Vorschriften der EP-A 58 055 können die Verbindungen der Beispiele 153 -160 erhalten werden.

Verbindungen der Formel I, $R^3$=$R^4$=$R^5$=H, B=$B^3$

| Beispiel | $R^1$ | $R^2$ | -A- | $R^9$ |
|---|---|---|---|---|
| 153 | $-(CH_2)_5-$ | | $-(CH_2)_4-$ | 3,4-Dimethoxyphenyl |
| 154 | $-(CH_2)_5-$ | | $-(CH_2)_4-$ | 3,4-Methylendioxophenyl |
| 155 | $-(CH_2)_5-$ | | $-(CH_2)_4-$ | 6-Methylpyridin-3-yl |
| 156 | $-(CH_2)_5-$ | | $-(CH_2)_5-$ | 6-Methylpyridin-3-yl |
| 157 | $C_6H_5CH_2$ | $CH_3$ | $-(CH_2)_4-$ | 6-Methylpyridin-3-yl |
| 158 | $C_6H_5CH_2$ | $CH_3$ | $-(CH_2)_5-$ | 6-Methylpyridin-3-yl |
| 159 | $n-C_4H_9$ | $n-C_4H_9$ | $-(CH_2)_4-$ | 6-Methylpyridin-3-yl |
| 160 | $n-C_4H_9$ | $n-C_4H_9$ | $-(CH_2)_5-$ | 6-Methylpyridin-3-yl |

Beispiele 161 -169

Verbindungen der Formel I, B = $B^4$

Durch Umsetzung der Verbindungen der Formel II mit 1,2-Dimethoxycyclobutendion zu Verbindungen der Formel X, $Y^4$ = $OCH_3$ und anschließende Umsetzung mit Aminen $R^{10}NH_2$ (in Analogie zu den Umsetzungen der BE 893 236) können die Verbindungen der Beispiele 161 -169 erhalten werden.

Verbindungen der Formel I, $R^3=R^4=R^5$, $B=B^4$

| Beispiel | $R^1$ | $R^2$ | -A- | $R^{10}$ |
|---|---|---|---|---|
| 161 | $-(CH_2)_5-$ | | $-(CH_2)_4-$ | H |
| 162 | $-(CH_2)_5-$ | | $-(CH_2)_4-$ | $CH_3$ |
| 163 | $C_6H_5CH_2$ | $CH_3$ | $-(CH_2)_4-$ | H |
| 164 | $C_6H_5CH_2$ | $CH_3$ | $-(CH_2)_4-$ | $CH_3$ |
| 165 | $n-C_4H_9$ | $n-C_4H_9$ | $-(CH_2)_4-$ | H |
| 166 | $n-C_4H_9$ | $n-C_4H_9$ | $-(CH_2)_4-$ | $CH_3$ |
| 167 | $-(CH_2)_5-$ | | $-(CH_2)_5$ | H |
| 168 | $C_6H_5CH_2$ | $CH_3$ | $-(CH_2)_5-$ | H |
| 169 | $n-C_4H_9$ | $n-C_4H_9$ | $-(CH_2)_5-$ | H |

Beispiele 170 -231

Verbindungen der Formel I, $B = B^5$

Analog Beispiel 27 können durch Umsetzung von Aminen der Formel II mit Verbindungen der Formel XI, wobei $Z^5$ den Rest NHR'' und $Y^5$ eine Methoxy-, Ethoxy-, 2-Methoxyethoxy-oder 2-Ethoxyethoxy-gruppe bedeutet, die Verbindungen der Beispiele 170 -231 erhalten werden.

Verbindungen der Formel I, $R^3=R^4=R^5=H$, $B=B^5$,

| Beispiel | $R^1$ | $R^2$ | -A- | m | $R^{11}$ |
|---|---|---|---|---|---|
| 170 | $-(CH_2)_5-$ | | $-(CH_2)_4-$ | 0 | H |
| 171 | $-(CH_2)_5-$ | | $-(CH_2)_4-$ | 0 | $CH_3$ |
| 172 | $-(CH_2)_5-$ | | $-(CH_2)_4-$ | 0 | (Pyridin-3-yl)-$CH_2$ |
| 173 | $-(CH_2)_5-$ | | $-(CH_2)_4-$ | 1 | (Pyridin-3-yl)-$CH_2$- |
| 174 | $-(CH_2)_5-$ | | $-(CH_2)_4-$ | 1 | (6-Methylpyridin-3-yl)-$CH_2$- |
| 175 | $-(CH_2)_5-$ | | $-(CH_2)_4-$ | 2 | H |
| 176 | $-(CH_2)_5-$ | | $-(CH_2)_4-$ | 2 | $CH_3$ |
| 177 | $C_6H_5CH_2$ | $CH_3$ | $-(CH_2)_4-$ | 0 | H |
| 178 | $C_6H_5CH_2$ | $CH_3$ | $-(CH_2)_4-$ | 0 | $CH_3$ |
| 179 | $C_6H_5CH_2$ | $CH_3$ | $-(CH_2)_4-$ | 1 | (6-Methylpyridin-3-yl)-$CH_2$- |
| 180 | $C_6H_5CH_2$ | $CH_3$ | $-(CH_2)_4-$ | 1 | (Pyridin-3-yl)-$CH_2$- |
| 181 | $C_6H_5CH_2$ | $CH_3$ | $-(CH_2)_4-$ | 2 | H |
| 182 | $-(CH_2)_5-$ | | $-(CH_2)_5-$ | 0 | H |
| 183 | $-(CH_2)_5-$ | | $-(CH_2)_5-$ | 0 | $CH_3$ |
| 184 | $-(CH_2)_5-$ | | $-(CH_2)_5-$ | 0 | (Pyridin-3-yl)-$CH_2$- |
| 185 | $-(CH_2)_5-$ | | $-(CH_2)_5-$ | 1 | (6-Methylpyridin-3-yl)-$CH_2$- |
| 186 | $-(CH_2)_5-$ | | $-(CH_2)_5-$ | 2 | H |

| Beispiel | $R^1$ | $R^2$ | -A- | m | $R^{11}$ |
|---|---|---|---|---|---|
| 187 | $-(CH_2)_5-$ | | $-(CH_2)_5-$ | 2 | $CH_3$ |
| 188 | $CH_3$ | $CH_3$ | $-(CH_2)_5-$ | 0 | (Pyridin-3-yl)-$CH_2$- |
| 189 | $CH_3$ | $CH_3$ | $-(CH_2)_5-$ | 1 | (6-Methylpyridin-3-yl)-$CH_2$- |
| 190 | $C_6H_5CH_2$ | $CH_3$ | $-(CH_2)_5-$ | 0 | H |
| 191 | $C_6H_5CH_2$ | $CH_3$ | $-(CH_2)_5-$ | 0 | (Pyridin-3-yl)-$CH_2$- |
| 192 | $C_6H_5CH_2$ | $CH_3$ | $-(CH_2)_5-$ | 1 | (6-Methylpyridin-3-yl)-$CH_2$- |
| 193 | $C_6H_5CH_2$ | $CH_3$ | $-(CH_2)_5-$ | 2 | H |
| 194 | n-$C_4H_9$ | n-$C_4H_9$ | $-(CH_2)_4-$ | 1 | $CH_3$ |
| 195 | n-$C_4H_9$ | n-$C_4H_9$ | $-(CH_2)_4-$ | 0 | H |
| 196 | n-$C_4H_9$ | n-$C_4H_9$ | $-(CH_2)_4-$ | 2 | H |
| 197 | n-$C_4H_9$ | n-$C_4H_9$ | $-(CH_2)_4-$ | 1 | (Pyridin-3-yl)-$CH_2$- |
| 198 | n-$C_4H_9$ | n-$C_4H_9$ | $-(CH_2)_4-$ | 1 | (6-Methylpyridin-3-yl)-$CH_2$ |
| 199 | n-$C_4H_9$ | n-$C_4H_9$ | $-(CH_2)_5-$ | 1 | H |
| 200 | n-$C_4H_9$ | n-$C_4H_9$ | $-(CH_2)_5-$ | 1 | $CH_3$ |
| 201 | n-$C_4H_9$ | n-$C_4H_9$ | $-(CH_2)_5-$ | 1 | (Pyridin-3-yl)-$CH_2$- |
| 202 | n-$C_4H_9$ | n-$C_4H_9$ | $-(CH_2)_5-$ | 1 | (6-Methyl-pyridin-3-yl)-$CH_2$- |
| 203 | n-$C_{12}H_{25}$ | H | $-(CH_2)_4-$ | 1 | H |
| 204 | n-$C_{12}H_{25}$ | H | $-(CH_2)_4-$ | 1 | $CH_3$ |

| Beispiel | $R^1$ | $R^2$ | -A- | m | $R^{11}$ |
|---|---|---|---|---|---|
| 205 | $n-C_{12}H_{25}$ | $CH_3$ | $-(CH_2)_4-$ | 1 | H |
| 206 | $n-C_{12}H_{25}$ | $CH_3$ | $-(CH_2)_4-$ | 1 | $CH_3$ |
| 207 | $n-C_{12}H_{25}$ | H | $-(CH_2)_5-$ | 1 | $CH_3$ |
| 208 | $n-C_{12}H_{25}$ | $CH_3$ | $-(CH_2)_5-$ | 1 | H |
| 209 | $n-C_{12}H_{25}$ | $CH_3$ | $-(CH_2)_5-$ | 1 | $CH_3$ |
| 210 | $pCH_3-C_6H_4CH_2$ | $CH_3$ | $-(CH_2)_5-$ | 1 | H |
| 211 | $pCl-C_6H_4-CH_2$ | $CH_3$ | $-(CH_2)_5-$ | 1 | H |
| 212 | $mCF_3-C_6H_4CH_2$ | $CH_3$ | $-(CH_2)_5-$ | 1 | H |
| 213 | $pNC-C_6H_4CH_2$ | $CH_3$ | $-(CH_2)_5-$ | 1 | H |
| 214 | 3-Thienyl-$CH_2$ | $CH_3$ | $-(CH_2)_5-$ | 1 | H |
| 215 | 5-Oxazolyl-$CH_2$ | $CH_3$ | $-(CH_2)_5-$ | 1 | H |
| 216 | 2-Thiazolyl-$CH_2$ | $CH_3$ | $-(CH_2)_5-$ | 1 | H |
| 217 | 3-Thienyl-$CH_2$ | $CH_3$ | $-(CH_2)_4-$ | 1 | H |
| 218 | $C_6H_5$<br>$-(CH_2)_2-C-(CH_2)_2-$<br>$COOC_2H_5$ | | $-(CH_2)_4-$ | 1 | H |
| 219 | $-(CH_2)_2-CH-(CH_2)_2-$<br>$C_2H_5CON-C_6H_5$ | | $-(CH_2)_4-$ | 1 | H |
| 220 | $-(CH_2)_2-CH-(CH_2)_2-$<br>$NH-CO-C_6H_5$ | | $-(CH_2)_4-$ | 1 | H |

| Beispiel | $R^1$ | | $R^2$ | -A- | m | $R^{11}$ |
|---|---|---|---|---|---|---|
| 221 | | $-(CH_2)_{11}-$ | | $-(CH_2)_4-$ | 1 | H |
| 222 | | 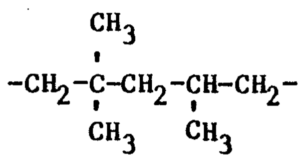 | | $-(CH_2)_4-$ | 1 | H |
| 223 | | $-(CH_2)_5-$ | | 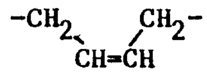 | 1 | H |
| 224 | | $-(CH_2)_5-$ | | 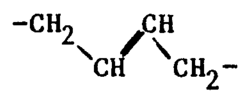 | 1 | H |
| 225 | $C_6H_5CH_2$ | | $CH_3$ | 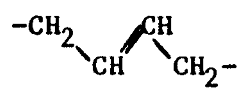 | 1 | H |
| 226 | $C_6H_5CH_2$ | | $CH_3$ | $-CH_2-C\equiv C-CH_2-$ | 1 | H |
| 227 | $C_6H_5CH_2$ | | $CH_3$ | $-(CH_2)_2S-(CH_2)_2-$ | 1 | H |
| 228 | $C_6H_5CH_2$ | | $CH_3$ | $-(CH_2)_2S-(CH_2)_2-$ | 1 | $CH_3$ |
| 229 | $C_6H_5CH_2$ | | $CH_3$ | $-(CH_2)_2S-(CH_2)_2-$ | 1 | (Pyridin-3-yl)-$CH_2$- |
| 230 | | $-(CH_2)_5-$ | | $-(CH_2)_2S-(CH_2)_2-$ | 1 | (Pyridin-3-yl)-$CH_2$- |
| 231 | | $-(CH_2)_5-$ | | $-(CH_2)_2S-(CH_2)_2-$ | 1 | (6-Methylpyridin-3-yl)-$CH_2$- |

Beispiele 232 -243

Verbindungen der Formel I, $B = B^6$

Analog Beispiel 86 können durch Umsetzung von 3-Chlorbenzoisothiazol-S,S-dioxiden bzw. der entsprechenden Thienoverbindungen mit den Aminen der Formel II die Verbindungen der Beispiele 232 -243 erhalten werden.

Verbindungen der Formel I, $R^3=R^4=R^5=H$, $B=B^6$

| Beispiel | $R^1$ | $R^2$ | -A- | $B^6$ |
|---|---|---|---|---|
| 232 | $-(CH_2)_5-$ | | $-(CH_2)_4-$ | 1) |
| 233 | $-(CH_2)_5-$ | | $-(CH_2)_4-$ | 2) |
| 234 | $C_6H_5CH_2$ | $CH_3$ | $-(CH_2)_4-$ | 2) |
| 235 | $C_6H_5CH_2$ | $CH_3$ | $-(CH_2)_4-$ | 3) |
| 236 | $n-C_4H_9$ | $n-C_4H_9$ | $-(CH_2)_4-$ | 1) |
| 237 | $n-C_4H_9$ | $n-C_4H_9$ | $-(CH_2)_4-$ | 2) |
| 238 | $-(CH_2)_5-$ | | $-(CH_2)_5-$ | 1) |
| 239 | $-(CH_2)_5-$ | | $-(CH_2)_5-$ | 2) |
| 240 | $C_6H_5CH_2$ | $CH_3$ | $-(CH_2)_5-$ | 1) |
| 241 | $C_6H_5CH_2$ | $CH_3$ | $-(CH_2)_5-$ | 2) |
| 242 | $n-C_4H_9$ | $n-C_4H_9$ | $-(CH_2)_5-$ | 1) |
| 243 | $n-C_4H_9$ | $n-C_4H_9$ | $-(CH_2)_5-$ | 2) |

1) $B^6$ = 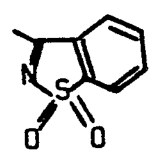 2) $B^6$ = 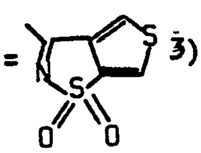 3) $B^6$ = 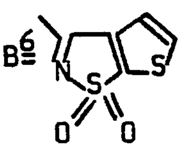

## Ansprüche

1. Verbindungen der allgemeinen Formel I

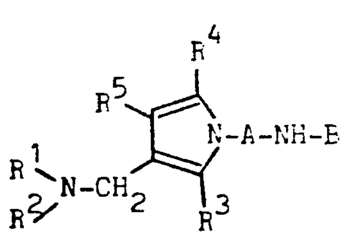 I,

worin

$R^1$ und $R^2$ unabhängig voneinander ein Wasserstoffatom oder einen $C_1$-$C_{12}$-Alkyl-, $C_3$-$C_{12}$-Alkenyl-oder $C_3$-$C_{12}$-Cycloalkylrest, die durch einen Phenyl-oder Naphthylrest, welche ihrerseits durch 1 oder 2 $C_{1-3}$-Alkyl-, $C_{1-3}$-Alkoxy-gruppen oder durch 1 bis 2 Fluor-, Chlor-oder Bromatome oder durch eine Trifluormethyl-, Carboxy-, ($C_{1-5}$-Alkoxy)-carbonyl-, Cyan-oder $C_{1-4}$-Acylamidogruppe substituiert sein können, oder durch einen 5gliedrigen aromatischen Ring mit 1 bis 3 Stickstoffatomen oder einem Sauerstoff-oder Schwefelatom und ggf. 1-2 Stickstoffatomen, oder durch einen 6gliedrigen aromatischen Ring mit 1 bis 3 Stickstoffatomen, wobei diese heteroaromatischen Ringe ggf. 1 bis 3 $C_{1-3}$-Alkylgruppen tragen, substituiert sein können, bedeuten, oder $R^1$ und $R^2$ zusammen mit dem Stickstoffatom einen gesättigten heterocyclischen Ring mit 4 bis 12 Ringgliedern bilden, der durch eine Phenyl-und/oder eine Hydroxy-, eine $C_{1-4}$-Acyl-, eine ($C_{1-5}$-Alkoxy)-carbonyl-, eine Nitril-, eine N-Phenyl-N-($C_{1-4}$-Acyl)-amino-, eine N-Benzamidogruppe oder einen Benzimidazolin-2-on-1-yl-rest und/oder durch 1 bis 3 $C_{1-4}$-Alkylgruppen substituiert sein oder einen ankondensierten Benzolring tragen kann,

$R^3$, $R^4$ und $R^5$ unabhängig voneinander Wasserstoffatome oder $C_{1-3}$-Alkylgruppen bedeuten, wobei $R^4$ und $R^5$ gemeinsam mit der Doppelbindung auch einen Benzolring bedeuten können,

A einen $C_{2-8}$-Alkylenrest bedeutet, der einfach olefinisch oder acetylenisch ungesättigt sein kann und in dem ein Kohlenstoffatom durch ein Sauerstoff-, ein Schwefelatom oder eine Phenylengruppe ersetzt sein kann,

B eine der Gruppen $B^1$ bis $B^6$ bedeutet:

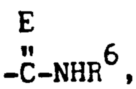,

$B^1$

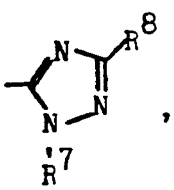,

$B^2$

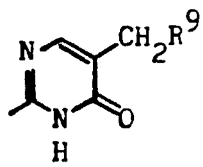

$B^3$

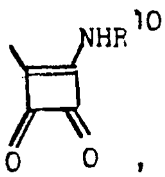,

$B^4$

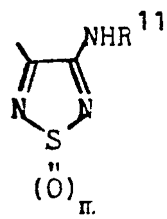

$B^5$

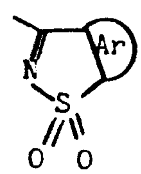

$B^6$

wobei

E S, $CH-NO_2$ oder N-CN bedeutet,

$R^6$ ein Wasserstoffatom oder einen $C_{1-3}$-Alkylrest, der durch einen 5gliedrigen aromatischen Ring mit 1 bis 3 Stickstoffatomen oder einem Sauerstoff-oder Schwefelatom und ggf. 1-2 Stickstoffatomen oder einen 6gliedrigen aromatischen Ring mit 1 bis 3 Stickstoffatomen, wobei diese heteroaromatischen Ringe ggf. 1 bis 3 $C_{1-3}$-Alkylgruppen tragen, oder durch eine Hydroxy-oder $C_{1-4}$ -Alkoxygruppe substituiert sein kann, bedeutet,

$R^7$ ein Wasserstoffatom oder einen $C_{1-3}$-Alkylrest bedeutet,

$R^8$ eine Aminogruppe oder den Rest $CH_2OR^{12}$ bedeutet, wobei

$R^{12}$ für ein Wasserstoffatom, einen $C_{1-3}$-Alkylrest, eine $C_{1-6}$-Acylgruppe, die Benzoylgruppe, den Tetrahydrofuran-2-yl oder Tetrahydropyran-2-yl-rest steht,

$R^9$ einen $C_{1-6}$-Alkylrest, eine Phenylgruppe, die durch je 1 oder 2 $C_{1-3}$-Alkyl-, $C_{1-3}$-Alkoxygruppen oder Chlor-oder Bromatome oder durch einen Methylendioxorest substituiert sein kann, oder einen 5gliedrigen aromatischen Ring mit 1 bis 3 Stickstoffatomen oder einem Sauerstoff-oder Schwefelatom und ggf. 1-2 Stickstoffatomen oder einen 6gliedrigen aromatischen Ring mit 1 bis 3 Stickstoffatomen, wobei diese heteroaromatischen Ringe ggf. 1 bis 3 $C_{1-3}$-Alkylgruppen tragen, bedeutet,

$R^{10}$ und $R^{11}$ Wasserstoffatome oder $C_{1-3}$-Alkylreste, die durch eine Phenylgruppe, die ihrerseits mit je 1 oder 2 $C_{1-3}$-Alkyl-, $C_{1-3}$-Alkoxygruppen oder Chlor-oder Bromatomen substituiert sein kann, oder durch einen 5gliedrigen aromatischen Ring mit 1 bis 3 Stickstoffatomen oder einem Sauerstoff-oder Schwefelatom und ggf. 1-2 Stickstoffatomen oder einem 6gliedrigen aromatischen Ring mit 1 bis 3 Stickstoffatomen, wobei diese heteroaromatischen Ringe ggf. 1 bis 3 $C_{1-3}$-Alkylgruppen tragen, oder durch eine Hydroxy-oder $C_{1-3}$ -Alkoxygruppe substituiert sein können, bedeuten,

m die Zahl 0, 1 oder 2 bedeutet und

Ar einen ankondensierten Benzol-oder Thiophenring darstellt, sowie deren physiologisch verträglichen Salze.

2. Verbindungen der allgemeinen Formel I, worin bedeuten:

$R^1$ und $R^2$ unabhängig voneinander ein Wasserstoffatom oder einen $C_{1-12}$-Alkyl-oder $C_3$-$C_{12}$-Cycloalkylrest, die durch einen Phenylrest substituiert sein können, wobei nicht beide Reste $R^1$ und $R^2$ gleichzeitig Wasserstoffatome bedeuten, oder

$R^1$ und $R^2$ zusammen mit dem Stickstoffatom einen gesättigten Ring mit 5 bis 10 Ringgliedern, der durch eine Phenylgruppe substituiert sein oder einen ankondensierten Benzolring enthalten kann,

$R^3$, $R^4$ und $R^5$ Wasserstoffatome oder Methylgruppen, wobei $R^4$ und $R^5$ gemeinsam mit der Doppelbindung einen Benzolring bilden können,

A einen $C_{3-6}$-Alkylenrest, der gegebenenfalls anstelle eines C-Atoms ein Schwefelatom als Kettenglied enthält,

B eine der Gruppen $B^1$, $B^2$, $B^5$ oder $B^6$,

E die Gruppe N-CN oder $CH-NO_2$,

$R^6$ einen $C_{1-3}$-Alkylrest, der gegebenenfalls durch eine Hydroxylgruppe substituiert ist,

$R^7$ ein Wasserstoffatom oder die Methylgruppe,

$R^8$ die Aminogruppe oder die Hydroxymethylgruppe,

$R^{11}$ ein Wasserstoffatom oder eine $C_{1-3}$-Alkylgruppe, die durch einen 5-oder 6gliedrigen heteroaromatischen Ring mit 1 bis 3 Stickstoffatomen substituiert sein kann, wobei dieser seinerseits ggf. durch 1 bis 3 $C_{1-3}$-Alkylgruppen substituiert ist,

m die Zahl 0 oder 1,

Ar einen ankondensierten Benzolring,

sowie deren physiologisch verträglichen Salze.

3. Verbindungen der allgemeinen Formel I, worin bedeuten:

$R^1$ eine $C_{1-12}$-Alkylgruppe oder eine Benzylgruppe, oder $R^1$ und $R^2$ zusammen mit dem Stickstoffatom einen 6-bis 7gliedrigen gesättigten Ring, der durch eine Phenylgruppe substituiert oder benzokondensiert sein kann,

$R^2$ ein Wasserstoffatom oder eine $C_{1-4}$-Alkylgruppe,

$R^3$, $R^4$ und $R^5$ Wasserstoffatome oder Methylgruppen,

A eine $C_{4-5}$-Alkylengruppe,

B eine der Gruppen $B^1$, $B^2$ oder $B^5$.

E die Gruppe $CH-NO_2$,

$R^6$ die Methylgruppe,

$R^7$ die Methylgruppe,

$R^8$ die Aminogruppe,

$R^{11}$ ein Wasserstoffatom, eine Methylgruppe oder eine Pyridylmethylgruppe,

m die Zahl 1,

sowie deren physiologisch verträglichen Salze.

4. Ein Amin der Formel II

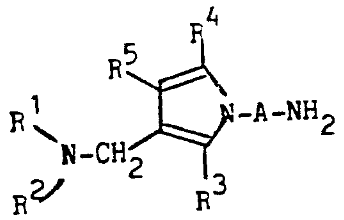

II,

wobei R' bis R⁵ und A dieselben Bedeutungen haben wie bei Formel I nach Anspruch 1.

5. Ein Amin der Formel II

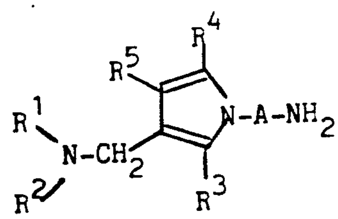

II,

wobei R' bis $R^5$ und A dieselben Bedeutungen haben wie bei Formel I nach Anspruch 2.

6. Ein Amin der Formel II

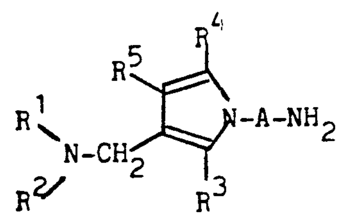

II,

wobei R' bis $R^5$ und A dieselben Bedeutungen haben wie bei Formel I nach Anspruch 3.

7. Therapeutisches Mittel, das neben den üblichen galenischen Hilfsmitteln eine Verbindung der allgemeinen Formel I nach Anspruch 1 als Wirkstoff enthält.